# EUROPEAN PATENT APPLICATION

(11) **EP 4 417 695 A2**
(43) Date of publication of application: **21.08.2024**
(21) Application number: 24180516.7
(22) Date of filing: 26.08.2020
(51) Int. Cl.: C12N 15/63

(54) **MODIFIED CIRCULAR RNAS AND METHODS OF USE THEREOF**

(30) Priority: 28.08.2019 US 201962892776 P
(62) Divisional of application: 20859564.5
(71) Applicant: The Board of Trustees of the Leland Stanford Junior University, Stanford, CA 94305-2038 (US)
(72) Inventor: CHANG, Howard Y., Stanford, CA 94305-2038 (US); CHEN, Robert, Stanford, CA 94305-2038 (US); AMAYA, Laura, Stanford, CA 94305-2038 (US); CHEN, Chun-Kan, Stanford, CA 94305-2038 (US)
(74) Representative: Algemeen Octrooi- en Merkenbureau B.V.

(57) **Abstract**

Provided herein are methods of generating a recombinant circular RNA molecule that comprises at least one N6-methyladenosine (m⁶A). The m⁶A-modified circRNA may be used to deliver a substance to a cell and to sequester an RNA-binding protein in a cell. Methods for modulating the immunogenicity of a circular RNA also are provided.

## Description

### CROSS-REFERENCE TO RELATED APPLICATIONS

This application claims the benefit of priority to U.S. Provisional Application No. 62/892,776, filed on August 28, 2019, which is hereby incorporated by reference in its entirety.

### STATEMENT REGARDING SEQUENCE LISTING

The Sequence Listing associated with this application is provided in text format in lieu of a paper copy, and is hereby incorporated by reference into the specification. The name of the text file containing the Sequence Listing is STDU2_37833_101_SeqList_ST25.txt. The file is ~4kb, was created on August 24, 2020, and is being submitted electronically.

### FIELD

The present application relates to methods of modifying circular RNA to reduce or increase the immunogenicity thereof, as well as methods of using the modified circular RNA.

### BACKGROUND

Tens of thousands of circular RNAs (circRNAs) have been identified in eukaryotes. Viruses like the hepatitis delta virus and plant viroids possess circRNA genomes, and many viruses produce circular RNAs as a normal part of their replication cycle. Recent studies suggest an emerging picture of an innate immune system based in part on circRNAs. Introduction of certain exogenous circRNAs can activate an antiviral and immune gene expression program, while endogenous circRNAs can collectively inhibit protein kinase R and set the threshold for innate immunity upon virus infection.

The mammalian innate immune system depends on pattern recognition receptors (PRRs) recognizing pathogen-associated molecular patterns (PAMPs) that are common among viruses and bacteria. RIG-I and MDA5 are PRRs found in the cytosol that sense foreign nucleic acids. MDA5 is known to detect long dsRNA whereas RIG-I has been shown to recognize 5' triphosphate on short dsRNAs. Although linear RNA ligands for RIG-I activation have been extensively characterized, RIG-I interaction with circRNAs has not been investigated, especially in the context of foreign circRNA detection.

N6-methyladenosine (m⁶A) is one of the most abundant RNA modifications. On mRNAs, m⁶A has been demonstrated to regulate different functions including splicing, translation, and degradation, which can have cell- and tissue-wide effects. Previous studies have suggested that m⁶A is also present on circRNA, and has the potential to initiate cap-independent translation. However, the effect of m⁶A on circRNA function and its role in RIG-I detection of circRNAs are not known.

There remains a need for compositions and methods to manipulate the immunogenicity of circular RNA, in order to use the circular RNA platform in biotechnology.

### BRIEF SUMMARY OF THE INVENTION

Provided herein are compositions and methods for manipulating the immunogenicity of circular RNA, and uses thereof.

In some embodiments, the disclosure provides a vaccine composition comprising a circular RNA molecule that does not contain any N6-methyladenosine (m⁶A) residues.

In some embodiments, the disclosure provides a composition comprising a DNA sequence coding a circular RNA, wherein the circular RNA does not contain any N6-methyladenosine (m⁶A) residues.

The disclosure also provides methods for eliciting an innate immune response in a subject in need thereof, the methods comprising administering to the subject an effective amount of a composition comprising a DNA sequence encoding a circular RNA as described herein.

The disclosure also provides methods for eliciting an innate immune response in a subject in need thereof, the methods comprising administering to the subject an effective amount of a vaccine composition comprising a circular RNA molecule that does not contain any m⁶A residues.

Also provided herein are methods for producing a circular RNA by *in vitro* transcription, the methods comprising providing a DNA template encoding the circular RNA molecule, ribonucleotide triphosphates, and a RNA polymerase; transcribing a linear RNA from the DNA template; and circularizing the linear DNA to form a circular RNA; wherein the ribonucleotide triphosphates do not include any N6-methyladenosine-5'-triphosphate (m⁶ATP); and wherein the circular RNA is capable of producing an innate immune response in the subject.

Also provided herein are methods for producing a circular RNA molecule by *in vitro* transcription, the methods comprising providing a DNA template encoding the circular RNA molecule, ribonucleotide triphosphates, and a RNA polymerase; transcribing a linear RNA from the DNA template; and circularizing the linear DNA to form a circular RNA; wherein the ribonucleotide triphosphates comprise N6-methyladenosine-5'-triphosphate (m⁶ATP); and wherein the circular RNA is less immunogenic compared to a circular RNA produced using the same method but in the absence of m⁶ATP.

The disclosure provides a method of delivering a substance to a cell, wherein the method comprises: (a) generating a recombinant circular RNA molecule that comprises at least one N6-methyladenosine (m⁶A); (b) attaching a substance to the recombinant circular RNA molecule to produce a complex comprising the recombinant circular RNA molecule attached to the substance; and (c) contacting a cell with the complex, whereby the substance is delivered to the cell.

The disclosure also provides a method of sequestering an RNA-binding protein in a cell, wherein the method comprises (a) generating a recombinant circular RNA molecule that comprises at least one N6-methyladenosine (m⁶A) and one or more RNA-binding protein binding domains; and (b) contacting a cell comprising the RNA-binding protein with the recombinant circular RNA molecule, whereby the RNA-binding protein binds to the one more RNA-binding protein binding domains and is sequestered in the cell.

The disclosure further provides a method of reducing the innate immunogenicity of a circular RNA molecule, wherein the method comprises: (a) providing a circular RNA molecule that induces an innate immune response in a subject; and (b) introducing at least one N6-methyladenosine (m⁶A) into the circular RNA molecule to provide a modified circular RNA molecule having reduced innate immunogenicity.

Also provided is a method of increasing the innate immunogenicity of a circular RNA molecule in a subject, wherein the method comprises: (a) generating a circular RNA molecule which lacks an RRACH motif (SEQ ID NO: 18); and (b) replacing one or more adenosines in the circular RNA sequence with another base (e.g., U, C, G, or inosine) to provide a modified circular RNA molecule having increased innate immunogenicity.

### BRIEF DESCRIPTION OF THE DRAWING(S)

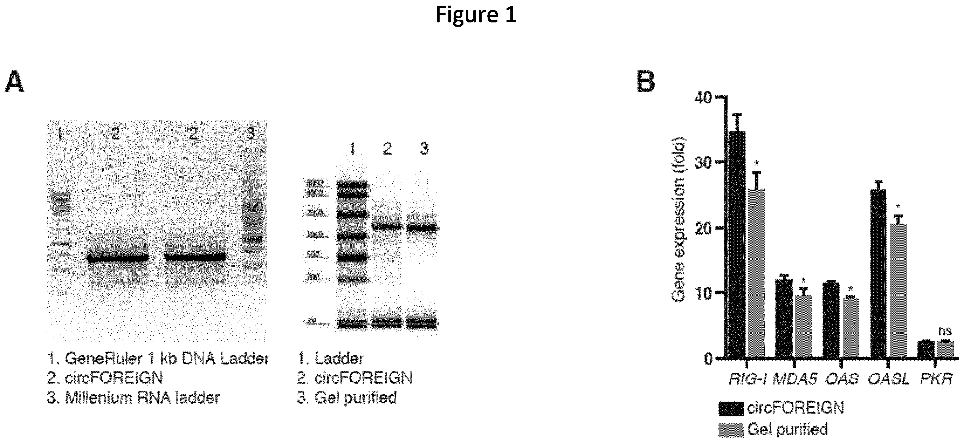

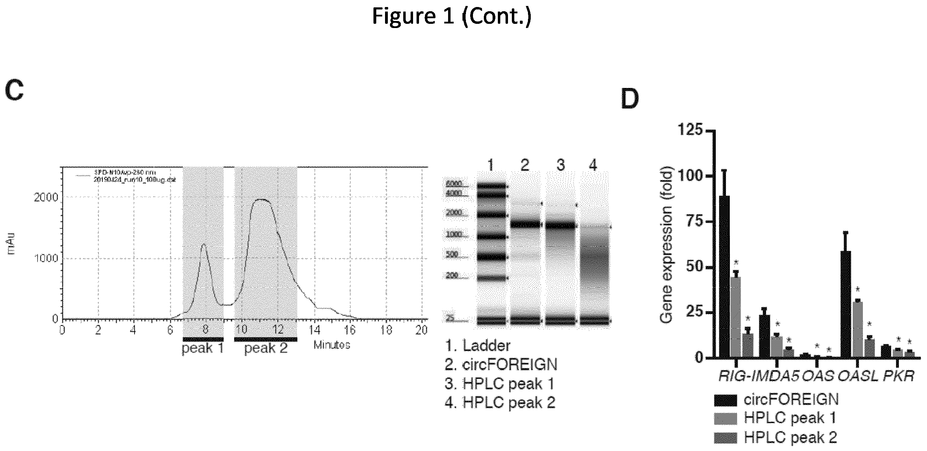

Figure 1A includes images depicting agarose gel electrophoresis of circFOREIGN prior to gel purification (left) and TapeStation analysis of resulting purified RNA (right). Figure 1B is a graph showing gene expression of innate immune genes 24 hours following RNA transfection into HeLa cells. Relative expression of the indicated mRNA and transfected RNA were measured by qRT-PCR, and results were normalized to expression following mock transfection. Means ± SEM are shown (n = 3). *p<0.05, Student's t-test, comparing circFOREIGN to gel purified RNA transfection. Figure 1C is a HPLC chromatogram of circFOREIGN purification. Collected fractions indicated on trace (left) and TapeStation analysis of purified RNA (right). Figure 1D is a graph showing gene expression of innate immune genes 24 hours following RNA transfection into HeLa cells. Relative expression of the indicated mRNA and transfected RNA were measured by qRT-PCR, and results were normalized to expression following mock transfection. Means ± SEM are shown (n = 3). *p<0.05, Student's t-test, comparing circFOREIGN to transfection with the indicated RNA.

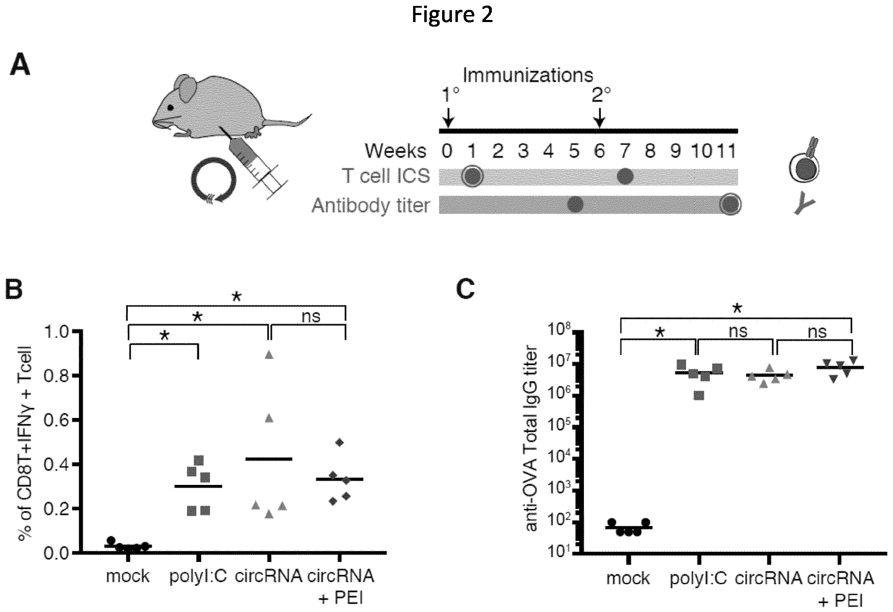

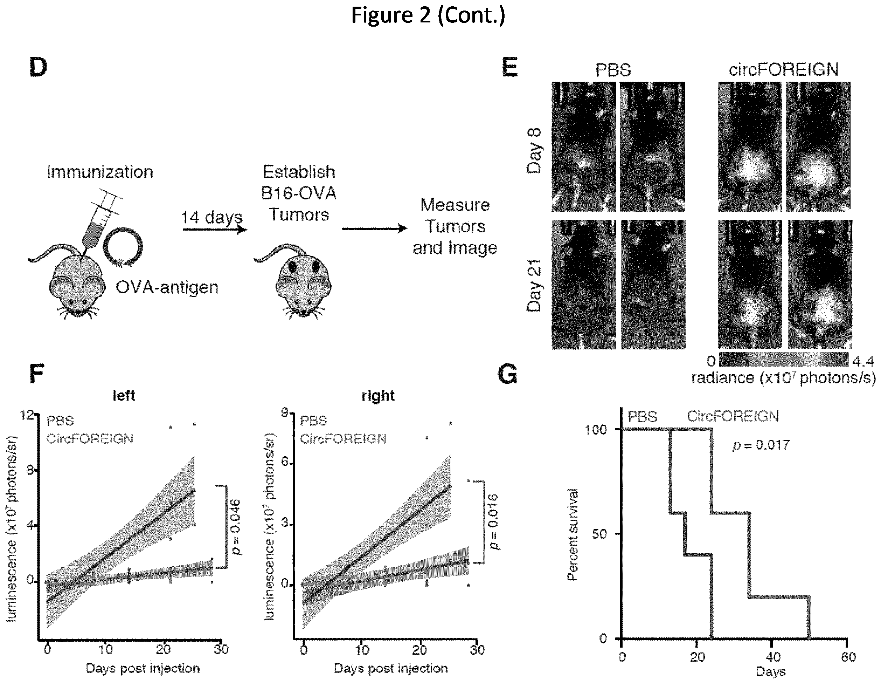

Figure 2A is a diagram depicting subcutaneous injection of agonist RNA in conjunction with OVA. T cell ICS and antibody titers were measured at the indicated times following primary and secondary immunizations. Figure 2B is a graph illustrating that circRNA stimulates anti-OVA T cell responses independent of transfection agent following primary vaccination. Means are shown (n = 5), *p < 0.05, Kruskal-Wallis test. Figure 2C is a graph illustrating that circRNA stimulates anti-OVA antibody titers independent of transfection agent following secondary vaccination. Means are shown (n = 5), *p < 0.05, Anova-Tukey's test. Figure 2D is a diagram depicting circFOREIGN vaccination in conjunction with OVA delivered by subcutaneous injection. 14 days later, OVA-expressing B16-melanoma cells were established in right and left flanks. Tumors were measured and imaged. Figure 2E includes images showing quantification of bioluminescence measurements in left and right tumors for mice vaccinated with PBS or circFOREIGN prior to tumor establishment. p value calculated by Wilcoxon signed-rank test. n=5 mice in each group. Figure 2F includes graphs showing quantification of bioluminescence measurements in left and right tumors for mice vaccinated with PBS or circFOREIGN prior to tumor establishment. p value calculated by Wilcoxon signed-rank test. n=5 mice in each group. Figure 2G is a graph showing that mice vaccinated with circFOREIGN survive twice as long as negative control mice. The graphs show survival curves for mice vaccinated with PBS or circFOREIGN prior to tumor establishment. p value calculated by log-rank test. n=5 mice in each group.

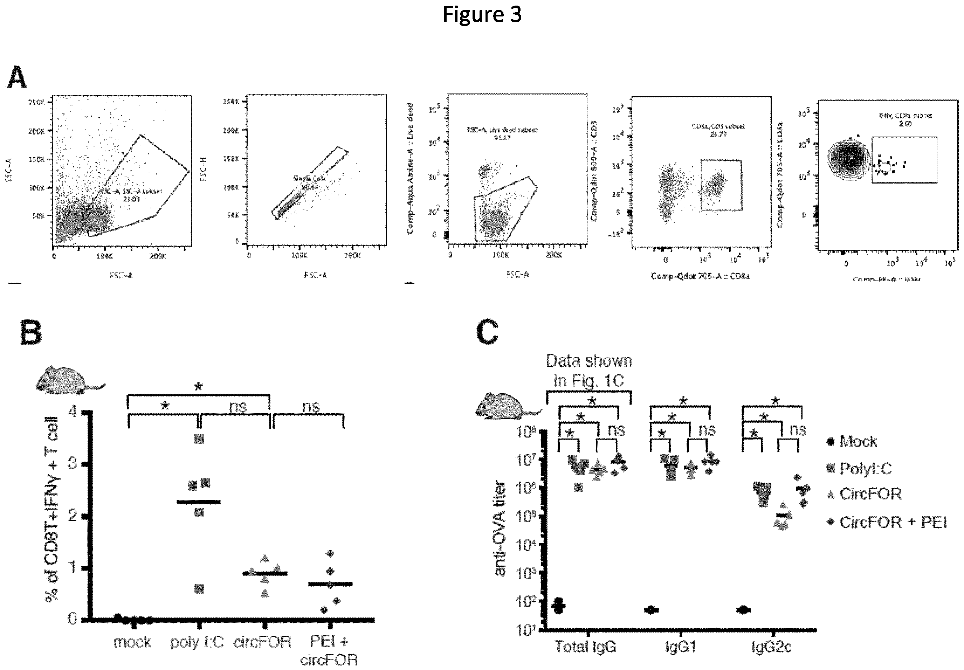

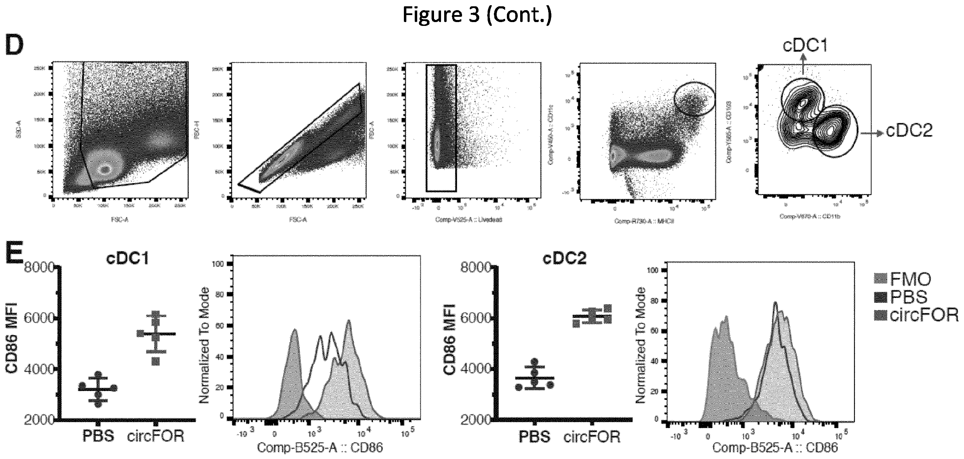

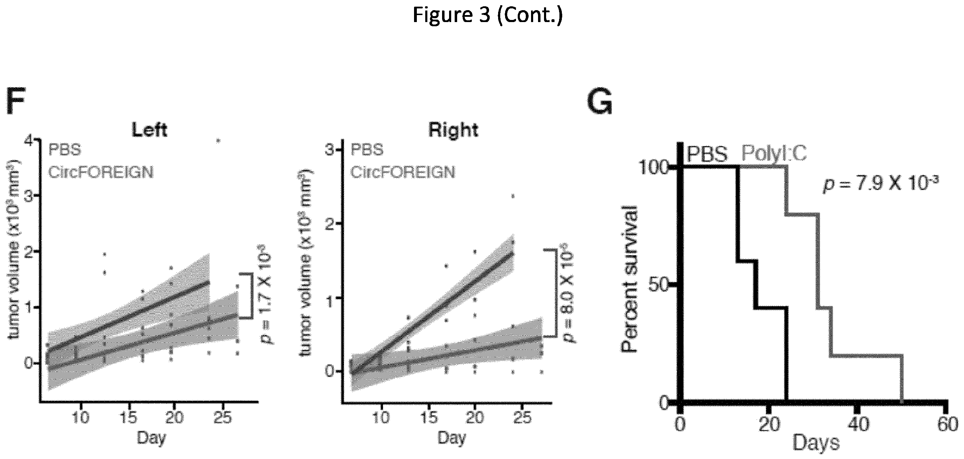

Figure 3A includes graphs showing gating strategy for FACS analysis of IFNγ + CD8 + T cells. Figure 3B is a graph showing that circFOREIGN stimulates anti-OVA specific T cell response independent of PE! after secondary immunization. Means are shown (n =5), *p<0.05, Anova-Tukey's test. Figure 3C is a graph showing that circFOREIGN stimulates anti-OVA antibody titers independent of PE! after secondary immunization. Means are shown (n = 5), *p<0.05, Anova-Tukey's test. Figure 3D includes graphs which show gating strategy for FACS analysis of cDC1 and cDC2 cells. Figure 3E includes graphs which illustrate that circFOREIGN immunization activates dendritic cells (DCs) in mice. Figure 3F includes graphs of measurements of left and right tumor volumes in mice vaccinated with PBS or circFOREIGN. p value calculated by Wilcoxon signed-rank test. Figure 3G includes graphs of survival curves of mice vaccinated with PBS or positive control polyl:C. p value calculated by log-rank test.

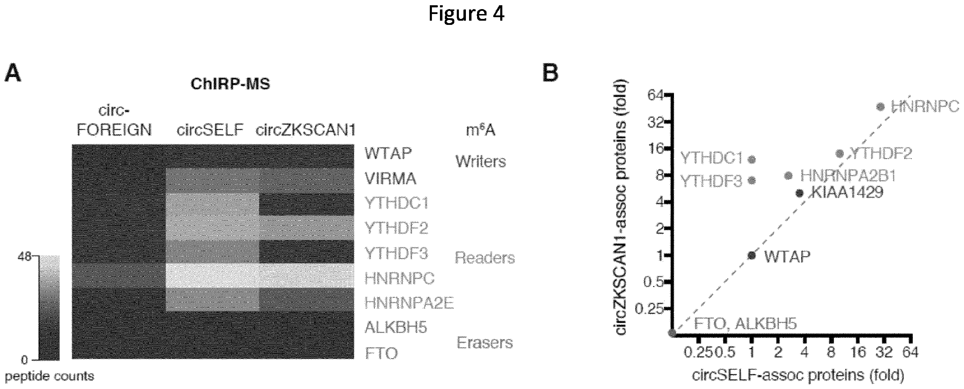

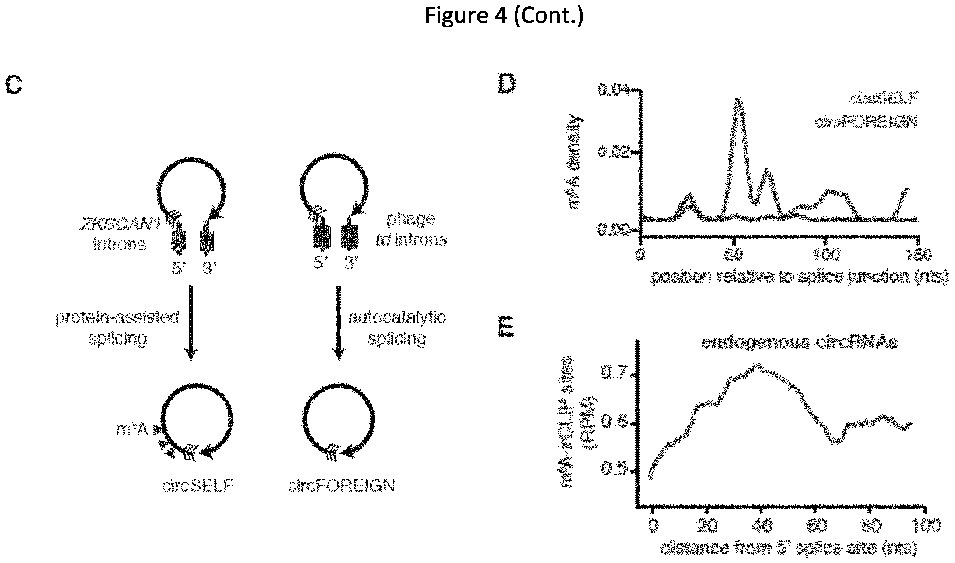

Figure 4A is a heatmap of peptide counts from ChIRP-MS of circZKSCAN1, circSELF, and circFOREIGN. Enzymes are classified as m⁶A writers, readers, and erasers. Figure 4B is a graph showing that m⁶A machinery associates with circZKSCAN1 and circSELF but not circFOREIGN, as indicated by ChIRP-MS. Fold enrichment over RNase-treated control is shown. Figure 4C is a schematic model showing ZKSCAN1 introns directing protein-assisted splicing to yield m⁶A-modified circSELF and phage td introns directing autocatalytic splicing to form unmodified circFOREIGN. Figure 4D is a graph showing that m⁶A-irCLIP identifies high-confidence m⁶A positions proximal to circRNA splice junctions. ZKSCAN1 introns suffice to direct m⁶A modification on circSELF compared with td intron-directed circFOREIGN. Density of m⁶A-irCLIP reads were normalized to reads per million. Figure 4E is a graph showing m⁶A-irCLIP read density near a circRNA splice junction of endogenous human circRNAs in HeLa cells. Density of m⁶A-irCLIP reads were normalized to reads per million for reads proximal to circRNA splice junctions.

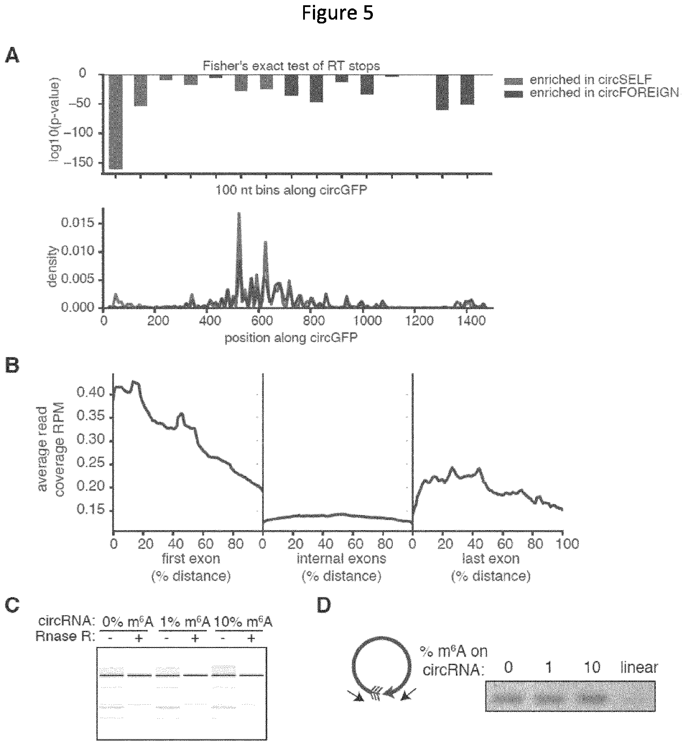

Figure 5A is a graph showing that m⁶A-irCLIP identifies high confidence m⁶A positions of circSELF or circFOREIGN. Fisher's exact test of RT stops enriched in circSELF or circFOREIGN are shown. Density of m⁶A-irCLIP reads were normalized to reads per million. Figure 5B is a graph showing m⁶A frequency on endogenous linear RNA. Figure 5C is an image showing TapeStation analysis *of in vitro* transcribed circFOREIGN with the indicated levels of m⁶A modification incorporated and with or without RNase R treatment. Figure 5D is an image of qRT-PCR over splice junctions confirming unmodified and m⁶A-modified circRNA formation during *in vitro* transcription. The figure shows an agarose gel of unmodified and m⁶A-modified circRNA after qRT-PCR using "inverted" primers as indicated.

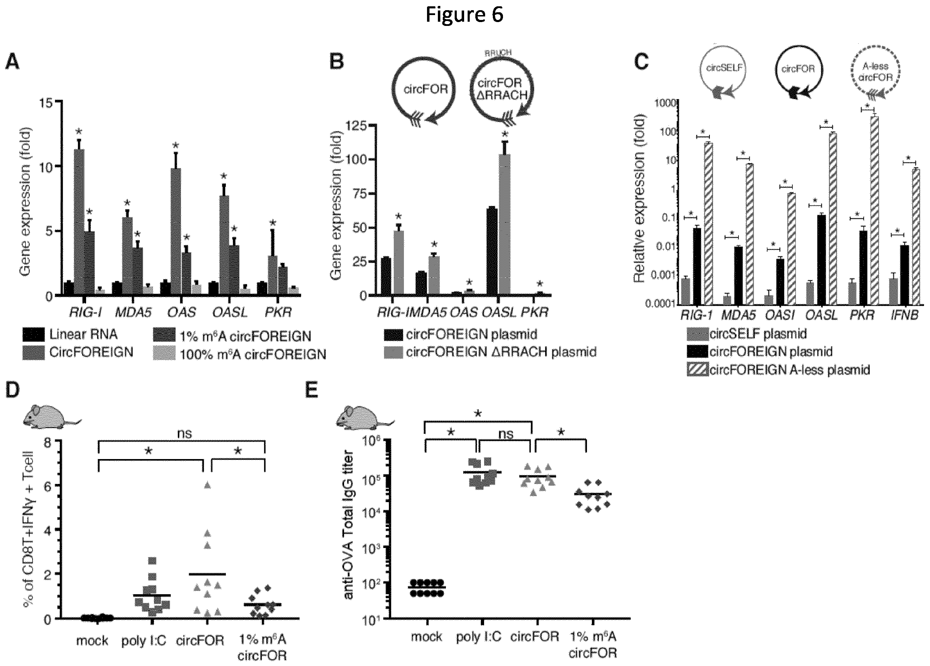

Figure 6A is a graph illustrating that transfection of unmodified circFOREIGN into wild-type HeLa cells stimulates an immune response, but m⁶A-modified circFOREIGN does not. The graph shows gene expression of innate immune genes 24 hours following RNA transfection. Relative expression of the indicated mRNA and transfected RNA were measured by qRT-PCR, and results were normalized to expression following mock transfection. Means ± SEM are shown (n = 3), *p < 0.05, Student's t-test, comparing gene stimulation of linear RNA to indicated RNA. Figure 6B is a graph illustrating that transfection of circFOREIGN plasmid lacking RRACH m⁶A consensus motifs (SEQ ID NO: 17) stimulates an immune response at a greater level than circFOREIGN. RRACH motifs (n = 12 sites) were mutated to RRUCH (SEQ ID NO: 19) throughout the exon sequence. Mutating every adenosine to uracil within the first 200 bases (n = 37 sites) after the splice junction further increased immunogenicity. The graph shows gene expression of innate immune genes following DNA plasmid transfection. Relative expression of the indicated mRNA and transfected RNA were measured by qRT-PCR, and results were normalized to expression following mock transfection. Means ± SEM are shown (n = 3), **p < 0.01, ***p < 0.001, Student's t-test, comparing circFOREIGN to transfection with the indicated RNA. Figure 6C is a graph illustrating that transfection of circFOREIGN plasmid with all adenosines replaced by uracil results in elevated immunogenicity. Relative expression of the indicated mRNA and transfected RNA were measured by qRT-PCR, and results were normalized to expression following mock transfection. Means ± SEM are shown (n = 3), *p<0.01, Student's t-test, comparing circFOREIGN to indicated RNA transfection. Figure 6D is a graph showing that m⁶A-modified circFOREIGN attenuates anti-OVA T cell responses following primary vaccination. Means are shown (n = 10), *p < 0.05, Anova-Tukey's test. Figure 6E is a graph showing that m⁶A-modified circRNA attenuates anti-OVA antibody titers following secondary vaccination. Means are shown (n = 10), *p < 0.05, ANOVA-Tukey's test.

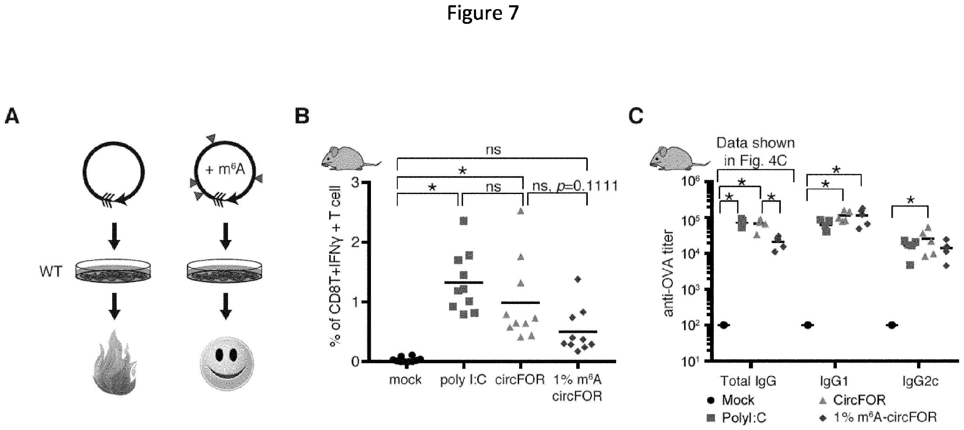

Figure 7A is a schematic model of unmodified and m⁶A-modified circFOREIGN effects on immunogenicity. Figure 7B is a graph showing that circFOREIGN stimulates an anti-OVA specific T cell response and 1% m⁶A-modifed circFOREIGN attenuates immunity after secondary immunization. Means are shown (n = 10), *p<0.05, Anova-Tukey's test. Figure 7C is a graph showing that circFOREIGN stimulates anti-OVA antibody titers and 1% m⁶A-modifed circRNA attenuates immunity after secondary immunization. Means are shown (n = 5), *p<0.05, Anova-Tukey's test.

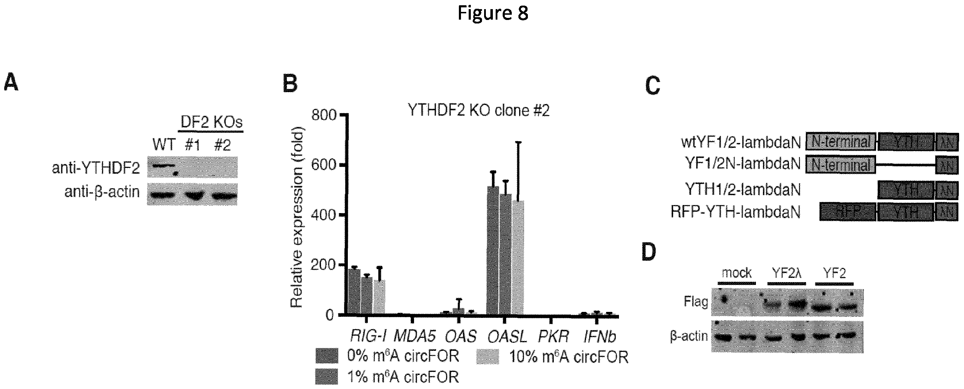

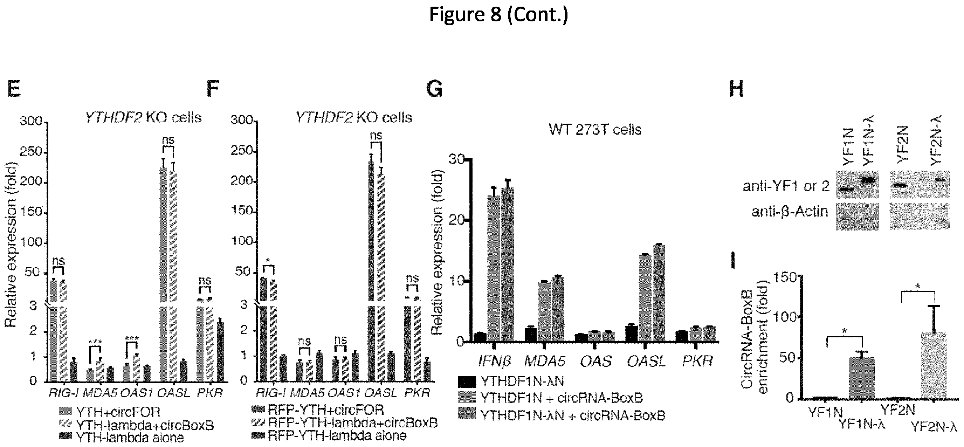

Figure 8A is an image of a Western blot of wild-type HeLa cells and two YTHDF2 knock-out (KO) clones. Figure 8B is a graph showing gene expression of innate immune genes 24 hours following RNA transfection into HeLa YTHDF2-/- clone #2. Relative expression of the indicated mRNA and transfected RNA are measured by qRT-PCR, and results were normalized to expression following mock transfection. Means ± SEM are shown (n = 3). Figure 8C is a schematic diagram of the YTHDF1/2 constructs used. Figure 8D is an image of Western blots of YTHDF2-λ, YTHDF2, YTHDF2N, YTHDF2N- λ, YTHDF1 N, and YTHDF1N-λ. Figure 8E is a graph showing RIP-qPCR enrichment of the indicated YTH protein followed by qRT-PCR of circRNA-BoxB or control actin RNA. Means ± SEM are shown (n = 3). *p<0.05, Student's t-test. Figure 8F is a graph showing that transfection of unmodified circBoxB tethered to the C-terminal YTH domain of YTHDF2 into YTHDF2 KO cells is insufficient to attenuate an immune response. Relative expression of the indicated mRNA and transfected RNA were measured by qRT-PCR, and results were normalized to expression following mock transfection. Means ± SEM are shown (n = 3). *p < 0.05, Student's t-test, comparing cells receiving +/- YTHDF2 transfection. Figure 8G is a graph showing that transfection of unmodified circBoxB tethered to RFP-YTH domain protein fusion into YTHDF2 KO cells is insufficient to attenuate an immune response. Relative expression of the indicated mRNA and transfected RNA were measured by qRT-PCR, and results were normalized to expression following mock transfection. Means ± SEM are shown (n = 3). *p < 0.05, Student's t-test, comparing cells receiving +/- YTHDF2 transfection. Figure 8H is a graph showing that transfection of unmodified circBoxB tethered to YTHDF1 is insufficient to attenuate an immune response. The graph shows gene expression of innate immune genes 24 hours following RNA transfection into wild-type HEK 293T cells. Relative expression of the indicated mRNA and transfected RNA were measured by qRT-PCR, and results were normalized to expression following transfection of plasmid expressing YTHDF1N-λN. Means ± SEM are shown (n = 3).

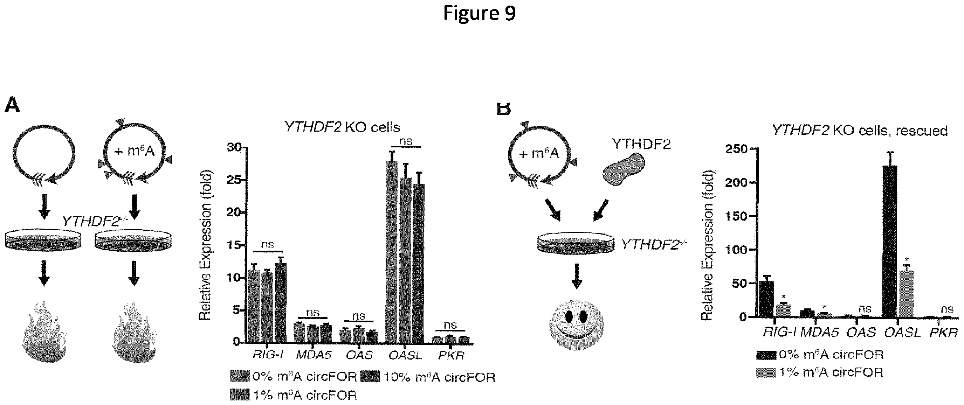

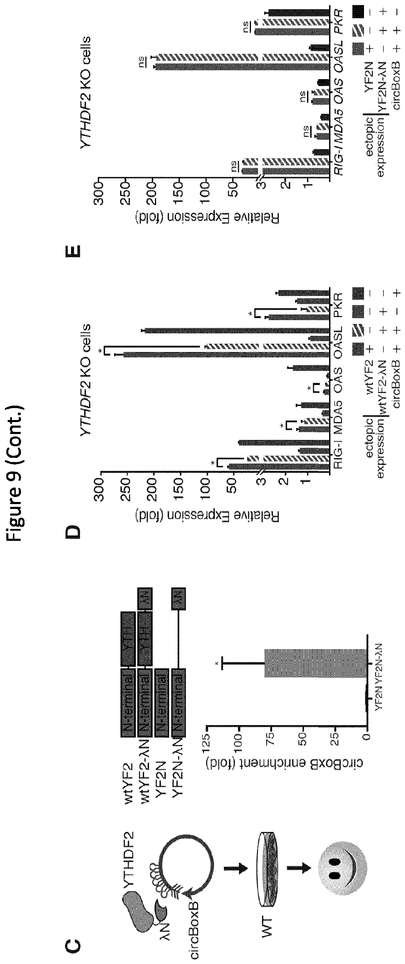

Figure 9A includes a schematic model showing the responses to unmodified or m⁶A-modified circFOREIGN. Transfection of unmodified or m⁶A-modified circFOREIGN into YTHDF2-/- HeLa cells stimulated an immune response. The right panel of Figure 9A is a graph showing gene expression of innate immune genes 24 hours following RNA transfection. Relative expression of the indicated mRNA and transfected RNA were measured by qRT-PCR, and results were normalized to expression following mock transfection. Means ± SEM are shown (n = 3). Student's t-test, comparing circFOREIGN with 0% m⁶A to indicated RNA transfection was used. Figure 9B shows that ectopic expression of YTHDF2 rescues the response to unmodified vs. m⁶A-modified circFOREIGN in YTHDF2 KO HeLa cells. The left panel of Figure 9B is a schematic model showing the response to m⁶A-modified circFOREIGN following rescue. The right panel of Figure 9B is a graph showing gene expression of innate immune genes 24 hours following RNA transfection. Relative expression of the indicated mRNA and transfected RNA were measured by qRT-PCR, and were normalized to expression following mock transfection. Means ± SEM are shown (n = 3). *p<0.05 using Student's t-test, comparing 0% m⁶A circFOREIGN to 1% m⁶A circFOREIGN. Figure 9C illustrates that tethering of YTHDF2 to unmodified circFOREIGN masks circRNA immunity. The left panel of Figure 9C is a schematic model showing *in vivo* tethering of protein to RNA via lambdaN and BoxB leading to attenuation of immunogenicity. The right top panel of Figure 9C is a diagram showing protein domain architecture of full-length wild-type YTHDF2 with and without a lambdaN tethering tag, and YTHDF2 N-terminal domain with and without the lambdaN tethering tag. The right bottom panel of Figure 9C is a graph showing RIP-qPCR enrichment of the indicated YTH protein followed by qRT-PCR of circRNA-BoxB or control actin RNA. Means ± SEM are shown (n = 3). *p<0.05 using Student's t-test, comparing YTHDF2 N-terminus with lambdaN tethering to YTHDF2 N-terminus without tethering. Figure 9D is a graph showing that transfection of unmodified circBoxB tethered to full length wild-type YTHDF2 into wild-type HeLa cells attenuated the immune response. The graph shows gene expression of innate immune genes 24 hours following RNA transfection. Relative expression of the indicated mRNA and transfected RNA were measured by qRT-PCR, and results were normalized to mock transfection. Wild-type YTHDF2-lambdaN (grey) was ectopically expressed as an immunogenicity negative control. Transfection with solely circBoxB served as an immunogenicity positive control. Means ± SEM are shown (n = 3). *p<0.05 using Student's t-test, comparing circBoxB with wild-type YTHDF2 with lambdaN tethering to wild-type YTHDF2 without tethering. Figure 9E is a graph showing that transfection of unmodified circBoxB tethered to the N-terminal domain of YTHDF2 into YTHDF2 KO cells is insufficient to attenuate the immune response. The graph shows gene expression of innate immune genes 24 hours following RNA transfection. Relative expression of the indicated mRNA and transfected RNA were measured by qRT-PCR, and results were normalized mock transfection. The N-terminal domain of YTHDF2-lambdaN (black) was ectopically expressed as an immunogenicity negative control. Means ± SEM are shown (n = 3). Student's t-test was used, comparing circBoxB with YTHDF2 N-terminus with lambdaN tethering to YTHDF2 N-terminus without tethering.

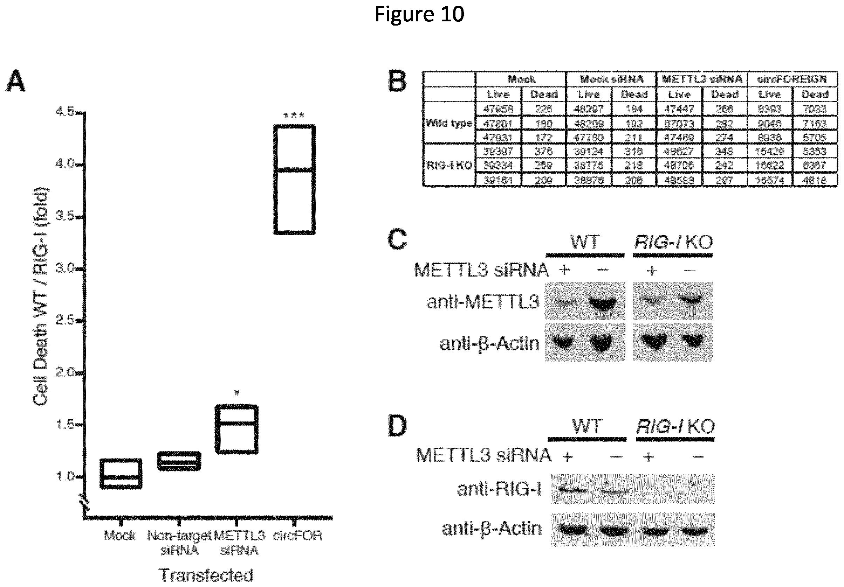

Figure 10A is a graph showing that RIG-I KO rescues cell death induced by depletion of m⁶A writer METTL3. The graph shows the fold change of cell death in wild-type or RIG-I KO HeLa cells following transfection of the indicated RNA. Means ± SEM are shown (n-50,000 cells analyzed). *p < 0.05, ***p<0.001 using Student's t-test, comparing mock transfection to indicated RNA transfection. Figure 10B is a table showing raw cell counts from the FACS analysis depicted in Figure 10A. Figure 10C is an image of Western blot validation of METTL3 knockdown efficiency in HeLa wild-type or RIG-I KO cells with METTL3 siRNA or non-targeting control siRNA transfection. Figure 10D is an image of Western blot validation of RIG-I protein expression in HeLa wild-type and RIG-I KO cells. Cells were transfected with METTL3 siRNA or non-targeting siRNA under comparable conditions to the FACS experiment.

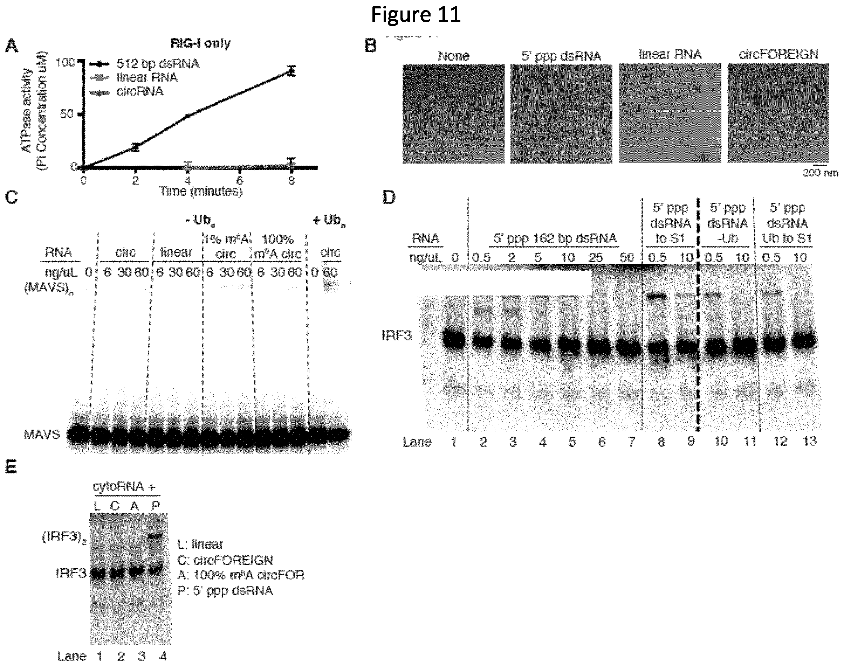

Figure 11A is a graph showing that circFOREIGN does not induce ATPase activity of RIG-I. RIG-I and RNA were incubated, and ATP was added. The reaction was quenched at the indicated time points and Pi concentration measured. Means ± SEM are shown (n = 2). Figure 11B includes representative electron microscopy images of RIG-I filaments after RIG-I was incubated with the indicated RNA. Figure 11C is an image depicting results of an *in vitro* RIG-I binding assay with purified RIG-I, K63-polyubiquitin, and the indicated RNA ligands. The depicted native electrophoretic gel shift assay shows that RIG-I binding does not distinguish between unmodified and m⁶A-modified circFOREIGN. Figure 11D is an image depicting results *of in vitro* reconstitution with purified RIG-I, MAVS, the indicated RNA ligands, and the absence or presence of K63-polyubiquitin. The depicted native gel of fluorescently-labeled MAVS 2CARD domain shows that circFOREIGN-initiated MAVS filamentation is dependent on K63-polyubiquitin. Figure 11E is an image showing *in vitro* reconstitution of the circRNA-mediated induction of IRF3 dimerization. RIG-I, IRF3, and the indicated RNA ligands were incubated. A native gel of radiolabeled-IRF3 with the indicated RNA ligands is shown. Cytoplasmic RNA (cytoRNA) and the indicated RNAs were each added at 0.5 ng/> L.

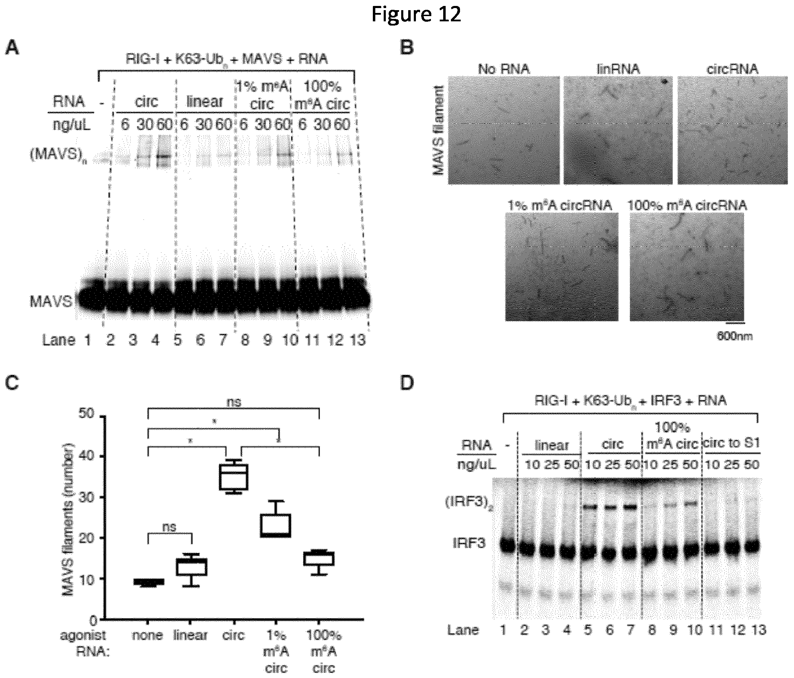

Figure 12A is an image depicting *in vitro* reconstitution with purified RIG-I, MAVS, K63-Ubn and the indicated RNA ligands. A native gel of fluorescently-labeled MAVS 2CARD domain is shown. Figure 12B includes representative electron microscopy images of MAVS filaments after MAVS polymerization assay with the indicated RNAs. Scale bar indicates 600 nm. Figure 12C is a graph showing quantification of the total number of MAVS filaments observed in five electron microscopy images for each agonist RNA. *p<0.05, Student's t-test. Figure 12D is an image depicting *in vitro* reconstitution of the circRNA-mediated induction of IRF3 dimerization. A native gel of radiolabeled-IRF3 with the indicated RNA ligands is shown. S1 is cellular extract.

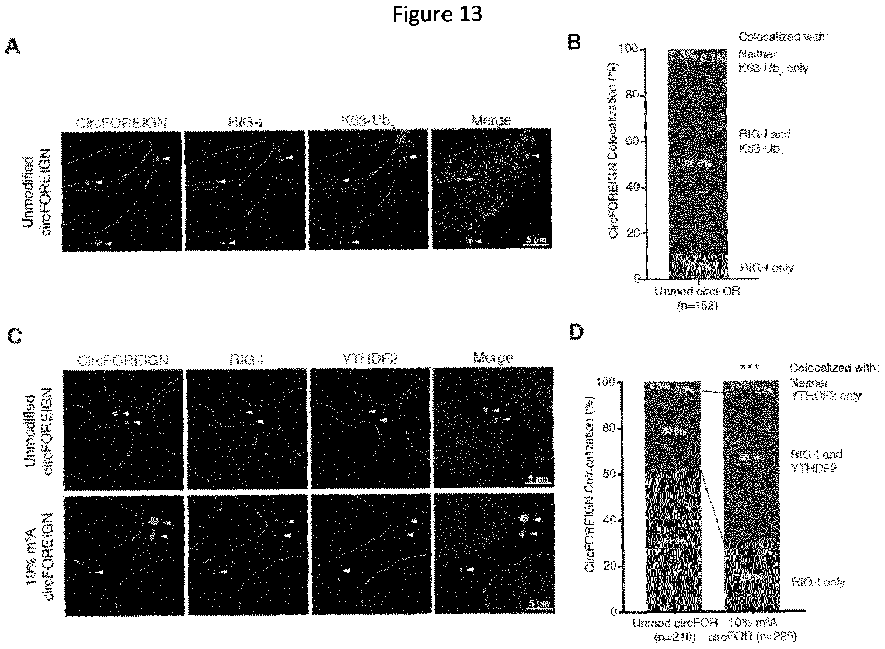

Figure 13A includes immunofluorescence images showing that circFOREIGN co-localizes with RIG-I and K63-polyubiquitin chain. Representative field of view is shown. Figure 13B is graph showing quantification of circFOREIGN colocalization with RIG-I and K63-Ubn (n = 152). Foci were collected across 10 fields of view across biological replicates and representative of replicate experiments. Figure 13C includes immunofluorescence images showing that 10% m⁶A circFOREIGN has increased co-localization with YTHDF2. Representative field of view is shown. Foci were collected across >10 fields of view and representative of replicate experiments. Figure 13D is a graph showing quantification of circFOREIGN and 10% m⁶A circFOREIGN colocalization with YTHDF2 and RIG-I. *p<0.05, Pearson's χ² test.

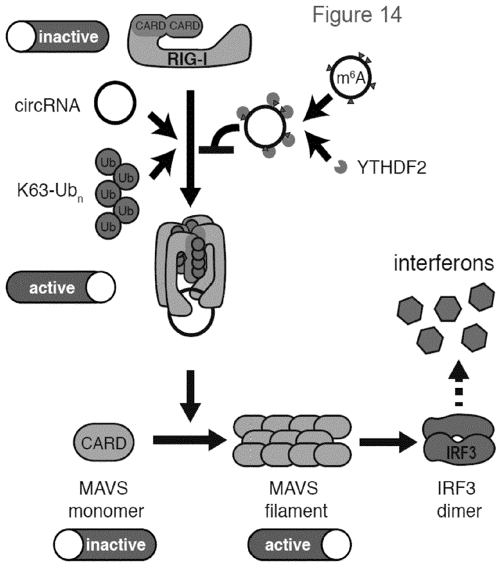

Figure 14 is a schematic diagram illustrating a proposed mechanism for RIG-I recognition of foreign circRNA that is dependent on K63-polyubiquitin.

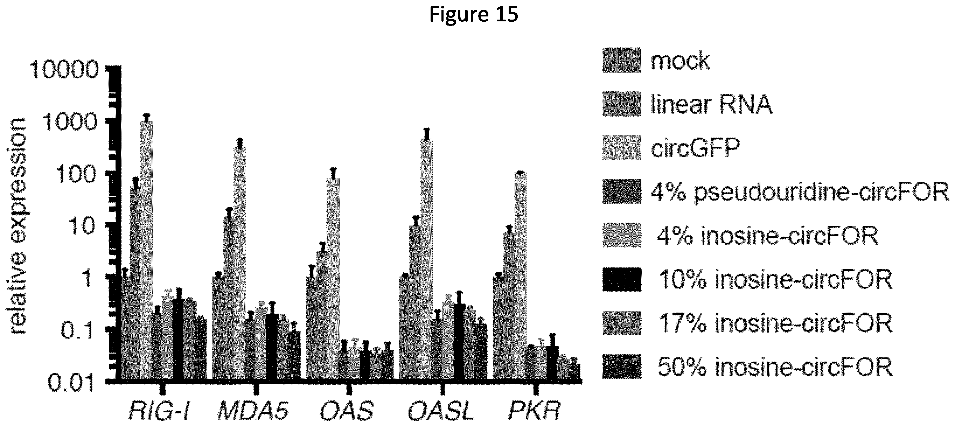

Figure 15 is a graph showing that transfection of unmodified circRNAs (i.e., lacking m⁶A modifications) into wild-type HeLa cells stimulate an immune response. The graph shows gene expression of innate immune genes 24 hours following RNA transfection. Relative expression of the indicated mRNA and transfected RNA were measured by qRT-PCR, results were normalized to expression following mock transfection. Means ± SEM are shown (n = 3).

### DETAILED DESCRIPTION OF THE INVENTION

The present disclosure is predicated, at least in part, on the discovery that N6-methyladenosine (m⁶A) RNA modification of human circular RNA molecules (circRNA) reduces the immunogenicity of circRNA. Foreign circRNAs are potent adjuvants that induce antigen-specific T cell activation, antibody production, and anti-tumor immunity *in vivo,* and the m⁶A modification thereof has been found to abrogate immune gene activation and adjuvant activity. The m⁶A reader protein YTHDF2 sequesters m⁶A-circRNA and is important for suppression of innate immunity.

### Definitions

To facilitate an understanding of the present technology, a number of terms and phrases are defined below. Additional definitions are set forth throughout the detailed description.

As used herein, the terms "nucleic acid," "polynucleotide," "nucleotide sequence," and "oligonucleotide" are used interchangeably and refer to a polymer or oligomer of pyrimidine and/or purine bases, preferably cytosine, thymine, and uracil, and adenine and guanine, respectively. The terms encompass any deoxyribonucleotide, ribonucleotide, or peptide nucleic acid component, and any chemical variants thereof, such as methylated, hydroxymethylated, or glycosylated forms of these bases. The polymers or oligomers may be heterogenous or homogenous in composition, may be isolated from naturally occurring sources, or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states. In some embodiments, a nucleic acid or nucleic acid sequence comprises other kinds of nucleic acid structures such as, for instance, a DNA/RNA helix, peptide nucleic acid (PNA), morpholino nucleic acid (see, e.g., Braasch and Corey, Biochemistry, 41(14): 4503-4510 (2002) and U.S. Patent 5,034,506), locked nucleic acid (LNA; see Wahlestedt et al., Proc. Natl. Acad. Sci. U.S.A., 97: 5633-5638 (2000)), cyclohexenyl nucleic acids (see Wang, J. Am. Chem. Soc., 122: 8595-8602 (2000)), and/or a ribozyme. The terms "nucleic acid" and "nucleic acid sequence" may also encompass a chain comprising non-natural nucleotides, modified nucleotides, and/or non-nucleotide building blocks that can exhibit the same function as natural nucleotides (e.g., "nucleotide analogs").

The term "nucleoside," as used herein, refers to a purine or pyrimidine base attached to a ribose or deoxyribose sugar. Nucleosides commonly found in DNA or RNA include cytidine, cytosine, deoxyriboside, thymidine, uridine, adenosine, adenine deoxyriboside, guanosine, and guanine deoxyriboside. The term "nucleotide," as used herein, refers to one of the monomeric units from which DNA or RNA polymers are constructed, which comprises a purine or pyrimidine base, a pentose, and a phosphoric acid group. The nucleotides of DNA are deoxyadenylic acid, thymidylic acid, deoxyguanilic acid, and deoxycitidylic acid. The corresponding nucleotides of RNA are adenylic acid, uridylic acid, guanylic acid, and citidylic acid.

The terms "peptide," "polypeptide," and "protein" are used interchangeably herein, and refer to a polymeric form of amino acids comprising at least two or more contiguous amino acids, which can include coded and non-coded amino acids, chemically or biochemically modified or derivatized amino acids, and polypeptides having modified peptide backbones.

Nomenclature for nucleotides, nucleic acids, nucleosides, and amino acids used herein is consistent with International Union of Pure and Applied Chemistry (IUPAC) standards (see, e.g., bioinformatics.org/sms/iupac.html).

As used herein, the term "RRACH motif" refers to a five nucleotide DNA or RNA motif, wherein R can be A or G, and H can be A, C, or T/U. RRACH motifs have a consensus sequence 5'-(A or G)-(A or G)-A-C-(A or C or T)-3' in DNA (SEQ ID NO: 17) or 5'-(A or G)-(A or G)-A-C-(A or C or U)-3' (SEQ ID NO: 18) in RNA. m⁶A modification typically occurs within an RRACH motif in eukaryotic cells. In many cell types, addition of m⁶A is catalyzed by a multicomponent methyltransferase complex, which includes METTL3, METTL14 and WTAP. In some embodiments, an RRACH motif (SEQ ID NO: 17-18) may be modified to reduce or eliminate m⁶A modifications. For example, an RRACH motif may be modified to a RRUCH motif (SEQ ID NO: 19-20).

An "antigen" is a molecule that triggers an immune response in a mammal. An "immune response" can entail, for example, antibody production and/or the activation of immune effector cells. An antigen in the context of the disclosure can comprise any subunit, fragment, or epitope of any proteinaceous or non-proteinaceous (e.g., carbohydrate or lipid) molecule that provokes an immune response in a mammal. By "epitope" is meant a sequence of an antigen that is recognized by an antibody or an antigen receptor. Epitopes also are referred to in the art as "antigenic determinants." The antigen can be a protein or peptide of viral, bacterial, parasitic, fungal, protozoan, prion, cellular, or extracellular origin, which provokes an immune response in a mammal, preferably leading to protective immunity.

The term "recombinant," as used herein, means that a particular nucleic acid (DNA or RNA) is the product of various combinations of cloning, restriction, polymerase chain reaction (PCR) and/or ligation steps resulting in a construct having a structural coding or noncoding sequence distinguishable from endogenous nucleic acids found in natural systems. DNA sequences encoding polypeptides can be assembled from cDNA fragments or from a series of synthetic oligonucleotides to provide a synthetic nucleic acid which is capable of being expressed from a recombinant transcriptional unit contained in a cell or in a cell-free transcription and translation system. Genomic DNA comprising the relevant sequences can also be used in the formation of a recombinant gene or transcriptional unit. Sequences of non-translated DNA may be present 5' or 3' from the open reading frame, where such sequences do not interfere with manipulation or expression of the coding regions, and may act to modulate production of a desired product by various mechanisms. Alternatively, DNA sequences encoding RNA that is not translated may also be considered recombinant. Thus, the term "recombinant" nucleic acid also refers to a nucleic acid which is not naturally occurring, e.g., is made by the artificial combination of two otherwise separated segments of sequence through human intervention. This artificial combination is often accomplished by either chemical synthesis means, or by the artificial manipulation of isolated segments of nucleic acids, e.g., by genetic engineering techniques. Such is usually done to replace a codon with a codon encoding the same amino acid, a conservative amino acid, or a non-conservative amino acid. Alternatively, the artificial combination may be performed to join together nucleic acid segments of desired functions to generate a desired combination of functions. This artificial combination is often accomplished by either chemical synthesis means, or by the artificial manipulation of isolated segments of nucleic acids, e.g., by genetic engineering techniques. When a recombinant polynucleotide encodes a polypeptide, the sequence of the encoded polypeptide can be naturally occurring ("wild type") or can be a variant (e.g., a mutant) of the naturally occurring sequence. Thus, the term "recombinant" polypeptide does not necessarily refer to a polypeptide whose sequence does not naturally occur. Instead, a "recombinant" polypeptide is encoded by a recombinant DNA sequence, but the sequence of the polypeptide can be naturally occurring ("wild type") or non-naturally occurring (e.g., a variant, a mutant, etc.). Thus, a "recombinant" polypeptide is the result of human intervention, but may comprise a naturally occurring amino acid sequence.

The term "binding domain" refers to a protein domain that is able to bind non-covalently to another molecule. A binding domain can bind to, for example, a DNA molecule (a DNA-binding protein), an RNA molecule (an RNA-binding protein) and/or a protein molecule (a protein binding protein). In the case of a protein domain-binding protein, the protein can bind to itself (to form homodimers, homotrimers, etc.) and/or it can bind to one or more molecules of a different protein or proteins.

### Circular RNAs

Circular RNAs (circRNAs) are single-stranded RNAs that are joined head to tail and were initially discovered in pathogenic genomes such as hepatitis D virus (HDV) and plant viroids. circRNAs have been recognized as a pervasive class of noncoding RNAs in eukaryotic cells. Generated through back splicing, circRNAs have been postulated to function in cell-to-cell information transfer or memory due to their extraordinary stability.

Although the functions of endogenous circRNAs are not known, their large number and the presence of viral circRNA genomes necessitate a system of circRNA immunity, as evidenced by the recent discoveries of human circRNA modulation of viral resistance through regulation of NF90/NF110 (Li et al., 2017) and autoimmunity through PKR regulation (Liu et al., 2019). As demonstrated herein, circRNAs can act as potent adjuvants to induce specific T and B cell responses. In addition, circRNA can induce both innate and adaptive immune responses and has the ability to inhibit the establishment and growth of tumors.

Because intron identity dictates circRNA immunity (Chen et al., *supra)* but is not part of the final circRNA product, it has been hypothesized that introns may direct the deposition of one or more covalent chemical marks onto circRNA. Among the over 100 known RNA chemical modifications, m⁶A is the most abundant modification on linear mRNAs and long noncoding RNAs, present on 0.2% to 0.6% of all adenosines in mammalian polyA-tailed transcripts (Roundtree et al., Cell, 169: 1187-1200 (2017)). m⁶A has recently been detected on mammalian circRNAs (Zhou et al., Cell Reports, 20: 2262-2276 (2017)). As described herein, human circRNAs appear to be marked at birth by one or more covalent m⁶A modifications, based on the introns that program their back splicing.

In some embodiments, the methods described herein involve generating a recombinant circular RNA molecule that comprises at least one N6-methyladenosine (m⁶A). Recombinant circRNA may be generated or engineered using routine molecular biology techniques. As disclosed above, recombinant circRNA molecules typically are generated by backsplicing of linear RNAs. In one embodiment, circular RNAs are produced from a linear RNA by backsplicing of a downstream 5' splice site (splice donor) to an upstream 3' splice site (splice acceptor). Circular RNAs can be generated in this manner by any non-mammalian splicing method. For example, linear RNAs containing various types of introns, including self-splicing group I introns, self-splicing group II introns, spliceosomal introns, and tRNA introns can be circularized. In particular, group I and group II introns have the advantage that they can be readily used for production of circular RNAs *in vitro* as well as *in vivo* because of their ability to undergo self-splicing due to their autocatalytic ribozyme activity.

Alternatively, circular RNAs can be produced *in vitro* from a linear RNA by chemical or enzymatic ligation of the 5' and 3' ends of the RNA. Chemical ligation can be performed, for example, using cyanogen bromide (BrCN) or ethyl-3-(3-dimethylaminopropyl) carbodiimide (EDC) for activation of a nucleotide phosphomonoester group to allow phosphodiester bond formation (Sokolova, FEBS Lett, 232:153-155 (1988); Dolinnaya et al., Nucleic Acids Res., 19: 3067-3072 (1991); Fedorova, Nucleosides Nucleotides Nucleic Acids, 15: 1137-1147 (1996)). Alternatively, enzymatic ligation can be used to circularize RNA. Exemplary ligases that can be used include T4 DNA ligase (T4 Dnl), T4 RNA ligase 1 (T4 Rnl 1), and T4 RNA ligase 2 (T4 Rnl 2).

In other embodiments, splint ligation may be used to circularize RNA. Splint ligation involves the use of an oligonucleotide splint that hybridizes with the two ends of a linear RNA to bring the ends of the linear RNA together for ligation. Hybridization of the splint, which can be either a deoxyribo-oligonucleotide or a ribooligonucleotide, orients the 5'-phosphate and 3'-OH of the RNA ends for ligation. Subsequent ligation can be performed using either chemical or enzymatic techniques, as described above. Enzymatic ligation can be performed, for example, with T4 DNA ligase (DNA splint required), T4 RNA ligase 1 (RNA splint required) or T4 RNA ligase 2 (DNA or RNA splint). Chemical ligation, such as with BrCN or EDC, is more efficient in some cases than enzymatic ligation if the structure of the hybridized splint-RNA complex interferes with enzymatic activity (see, e.g., Dolinnaya et al. Nucleic Acids Res, 21(23): 5403-5407 (1993); Petkovic et al., Nucleic Acids Res, 43(4): 2454-2465 (2015)).

Circular RNA molecules comprising one or more m⁶A modifications can be generated using any suitable method known in the art for introducing non-native nucleotides into nucleic acid sequences. In some embodiments, an m⁶A may be introduced into an RNA sequence using *in vitro* transcription methods, such as those described in, e.g., Chen et al., *supra.* An illustrative *in vitro* transcription reaction requires a purified linear DNA template containing a promoter, ribonucleotide triphosphates, a buffer system that includes DTT and magnesium, and an appropriate phage RNA polymerase (e.g., SP6, T7, or T3). As is understood by those of skill in the art, the exact conditions used in the transcription reaction depend on the amount of RNA needed for a specific application.

Any number of adenosines in a particular circRNA molecule generated as described herein may be modified (e.g., replaced) with a corresponding number of m⁶A's. Ideally, at least one adenosine in the circRNA molecule is replaced with an m⁶A. In some embodiments, at least 1% (e.g., 1%, 2%, 3%, 4%, 5%, 6%, 7%, 8%, 9% or more) of the adenosines in the recombinant circular RNA molecule are replaced with N6-methyladenosine (m⁶A). In other embodiments, at least 10% (e.g., 10%, 11%, 12%, 13%, 14%, 15%, 20%, 30%, 40%, 50%, 60%, 70%, 80%, 90%, or more) of the adenosines in the recombinant circular RNA molecule are replaced with N6-methyladenosine. For example, all (i.e., 100%) of the adenosines in the recombinant circular RNA molecule may be replaced with N6-methyladenosine (m⁶A). It will be appreciated that the number of m⁶A modifications introduced into a recombinant circular RNA molecule will depend upon the particular use of the circRNA, as described further herein.

In some embodiments, a method of producing a circular RNA molecule by *in vitro* transcription comprises providing a DNA template encoding the circular RNA molecule, ribonucleotide triphosphates, and a RNA polymerase; transcribing a linear RNA from the DNA template; and circularizing the linear DNA to form a circular RNA. In some embodiments, the ribonucleotide triphosphates do not include any N6-methyladenosine-5'-triphosphate (m⁶ATP). In some embodiments, the circular RNA is capable of producing an innate immune response in the subject. In some embodiments, the circular RNA is capable of producing an innate immune response in a subject.

In some embodiments, a method of producing a circular RNA molecule by *in vitro* transcription comprises providing a DNA template encoding the circular RNA molecule, ribonucleotide triphosphates, and a RNA polymerase; transcribing a linear RNA from the DNA template; and circularizing the linear DNA to form a circular RNA. In some embodiments, the ribonucleotide triphosphates comprise N6-methyladenosine-5'-triphosphate (m⁶ATP). In some embodiments, the circular RNA is less immunogenic compared to a circular RNA produced using the same method but in the absence of m⁶ATP. Immunogenicity of a circular RNA may be determined by measuring the inflammatory response after treatment with the circular RNA. In some embodiments, immunogenicity of a circular RNA may be determined by measuring the type I or type II interferon response, or the levels of one or more pro-inflammatory cytokines produced after treatment with the circular RNA. For example, immunogenicity of a circular RNA may be determined by measuring levels of levels of interferon alpha (IFNα), interferon beta (IFNβ), interferon gamma (IFNγ), interferon omega (IFNω), interleukin 1-beta (IL-1β), interleukin 6 (IL-6), tumor necrosis factor alpha (TNF-α), interleukin 12 (IL-12), interleukin 23 (IL-23), or interleukin-17 (IL-17) after circular RNA treatment. In some embodiments, immunogenicity may be determined by measuring expression or activity of one or more of retinoic acid inducible gene 1 (RIG-I), melanoma differentiation-associated protein 5 (MDA5), 2'-5'-oligoadenylate synthetase (OAS), OAS-like protein (OASL), and Double-stranded RNA-dependent protein kinase (PKR). Immunogenicity may be assessed *in vitro* or *in vivo.* A first circular RNA is less immunogenic than a second circular RNA if the inflammatory response after treatment with the first circular RNA is reduced compared to the inflammatory response after treatment with the second circular RNA.

In some embodiments, a circular RNA is designed to have a desired level of immunogenicity. For example, the circular RNA may be designed to be highly immunogenic, mildly immunogenic, substantially non-immunogenic, or non-immunogenic. The immunogenicity of a circular RNA may be controlled by modifying the number of RRACH motifs present in the circular RNA, wherein a greater number of RRACH motifs leads to reduced immunogenicity and a lower of RRACH motifs leads to increased immunogenicity. In some embodiments, a circular RNA or a DNA sequence encoding the same comprises 1-5, 5-10, 10-25, 25-100, 100-250, 250-500, or greater than 500 RRACH motifs.

In some embodiments, at least 1% of the adenosines in the recombinant circular RNA molecule are N6-methyladenosine (m⁶A). In some embodiments, at least 10% of the adenosines in the recombinant circular RNA molecule are N6-methyladenosine (m⁶A). In some embodiments, all of the adenosines in the recombinant circular RNA molecule are N6-methyladenosine (m⁶A).

In some embodiments, less than 1% of the adenosines in the recombinant circular RNA molecule are N6-methyladenosine (m⁶A). For example, less than 0.9%, less than 0.8%, less than 0.7%, less than 0.5%, less than 0.4%, less than 0.3%, less than 0.2%, or less than 0.1% of the adenosines in the recombinant circular RNA molecule may be m⁶A. In some embodiments, the recombinant circular RNA comprises 1-5, 5-10, 10-25, 25-100, 100-250, 250-500, or greater than 500 m⁶A residues.

While circular RNAs generally are more stable than their linear counterparts, primarily due to the absence of free ends necessary for exonuclease-mediated degradation, additional modifications may be made to the m⁶A-modified circRNA described herein to further improve stability. Still other kinds of modifications may improve circularization efficiency, purification of circRNA, and/or protein expression from circRNA. For example, the recombinant circRNA may be engineered to include "homology arms" (i.e., 9-19 nucleotides in length placed at the 5' and 3' ends of a precursor RNA with the aim of bringing the 5' and 3' splice sites into proximity of one another), spacer sequences, and/or a phosphorothioate (PS) cap (Wesselhoeft et al., Nat. Commun., 9: 2629 (2018)). The recombinant circRNA also may be engineered to include 2'-O-methyl-, -fluoro- or -O-methoxyethyl conjugates, phosphorothioate backbones, or 2',4'-cyclic 2'-O-ethyl modifications to increase the stability thereof (Holdt et al., Front Physiol., 9: 1262 (2018); Krützfeldt et al., Nature, 438(7068): 685-9 (2005); and Crooke et al., Cell Metab., 27(4): 714-739 (2018)).

In some embodiments, a circular RNA molecule comprises at least one intron and at least one exon. The term "exon," as used herein, refers to a nucleic acid sequence present in a gene which is represented in the mature form of an RNA molecule after excision of introns during transcription. Exons may be translated into protein (e.g., in the case of messenger RNA (mRNA)). The term "intron," as used herein, refers to a nucleic acid sequence present in a given gene which is removed by RNA splicing during maturation of the final RNA product. Introns are generally found between exons. During transcription, introns are removed from precursor messenger RNA (pre-mRNA), and exons are joined via RNA splicing.

In some embodiments, a circular RNA molecule comprises a nucleic acid sequence which includes one or more exons and one or more introns. In some embodiments, the circular RNA molecule one or more exons. In some embodiments, the circular RNA molecule does not comprise any introns.

In some embodiments, a circular RNA molecule may comprise an artificial sequence. The artificial sequence may confer favorable properties, such as desirable binding properties. For example, the artificial sequence may bind to one or more RNA binding proteins, or may be complementary to one or more micro RNAs. In some embodiments, the artificial sequence may be a scrambled version of a gene sequence or a sequence from a naturally occurring circular RNA. A scrambled sequence typically has the same nucleotide composition as the sequence from which it is derived. Methods for generating scrambled nucleic acids are known to those of skill in the art. In some embodiments, a circular RNA comprises an artificial sequence, but does not comprise an exon. In some embodiments, a circular RNA comprises an artificial sequence and also comprises at least one exon.

Accordingly, circular RNAs can be generated with either an endogenous or exogenous intron, as described in WO 2017/222911. Numerous intron sequences from a wide variety of organisms and viruses are known and include sequences derived from genes encoding proteins, ribosomal RNA (rRNA), or transfer RNA (tRNA). Representative intron sequences are available in various databases, including the Group I Intron Sequence and Structure Database (rna.whu.edu.cn/gissd/), the Database for Bacterial Group II Introns (webapps2.ucaigary.ca/-groupii/index.htm!), the Database for Mobile Group II Introns (fp.ucalgary.ca/group2introns), the Yeast Intron DataBase (emblS16 heidelberg.de/Externallnfo/seraphin/yidb.html), the Ares Lab Yeast Intron Database (compbio.soe.ucsc.edu/yeast_introns.html), the U12 Intron Database (genome.crg.es/cgibin/u12db/u12db.cgi), and the Exon-Intron Database (bpg.utoledo.edu/~afedorov/lab/eid.html).

In certain embodiments, the recombinant circular RNA molecule is encoded by a nucleic acid that comprises a self-splicing group I intron. Group I introns are a distinct class of RNA self-splicing introns which catalyze their own excision from mRNA, tRNA, and rRNA precursors in a wide range of organisms. All known group I introns present in eukaryote nuclei interrupt functional ribosomal RNA genes located in ribosomal DNA loci. Nuclear group I introns appear widespread among eukaryotic microorganisms, and the plasmodial slime molds (myxomycetes) contain an abundance of self-splicing introns. The self-splicing group I intron included in the circular RNA molecule may be obtained or derived from any suitable organism, such as, for example, bacteria, bacteriophages, and eukaryotic viruses. Self-splicing group I introns also may be found in certain cellular organelles, such as mitochondria and chloroplasts, and such self-splicing introns may be incorporated into a nucleic acid encoding the circular RNA molecule.

In certain embodiments, the recombinant circular RNA molecule is encoded by a nucleic acid that comprises a self-splicing group I intron of the phage T4 thymidylate synthase (td) gene. The group I intron of phage T4 thymidylate synthase (td) gene is well characterized to circularize while the exons linearly splice together (Chandry and Belfort, Genes Dev., 1: 1028-1037 (1987); Ford and Ares, Proc. Natl. Acad. Sci. USA, 91: 3117-3121 (1994); and Perriman and Ares, RNA, 4: 1047-1054 (1998)). When the td intron order is permuted (i.e., 5' half placed at the 3' position and vice versa) flanking any exon sequence, the exon is circularized via two autocatalytic transesterification reactions (Ford and Ares, *supra;* Puttaraju and Been, Nucleic Acids Symp. Ser., 33: 49-51 (1995)).

In some embodiments, the recombinant circular RNAs described herein may comprise an internal ribosome entry site (IRES), which may be operably linked to an RNA sequence encoding a polypeptide. Inclusion of an IRES permits the translation of one or more open reading frames from a circular RNA. The IRES element attracts a eukaryotic ribosomal translation initiation complex and promotes translation initiation (see, e.g., Kaufman et al., Nuc. Acids Res., 19: 4485-4490 (1991); Gurtu et al., Biochem. Biophys. Res. Comm, 229: 295-298 (1996); Rees et al., BioTechniques, 20: 102-110 (1996); Kobayashi et al., BioTechniques, 21: 399-402 (1996); and Mosser et al., BioTechniques, 22: 150-161 (1997)).

A number of IRES sequences are known in the art and may be included in a circular RNA molecule. For example, IRES sequences may be derived from a wide variety of viruses, such as from leader sequences of picornaviruses (e.g., encephalomyocarditis virus (EMCV) UTR) (Jang et al., J. Virol., 63: 1651-1660 (1989)), the polio leader sequence, the hepatitis A virus leader, the hepatitis C virus IRES, human rhinovirus type 2 IRES (Dobrikova et al., Proc. Natl. Acad. Sci., 100(25): 15125-15130 (2003)), an IRES element from the foot and mouth disease virus (Ramesh et al., Nucl. Acid Res., 24: 2697-2700 (1996)), and a giardiavirus IRES (Garlapati et al., J. Biol. Chem., 279(5): 3389-3397 (2004)). A variety of nonviral IRES sequences also can be included in a circular RNA molecule, including but not limited to, IRES sequences from yeast, the human angiotensin II type 1 receptor IRES (Martin et al., Mol. Cell Endocrinol., 212: 51-61 (2003)), fibroblast growth factor IRESs (e.g., FGF-1 IRES and FGF-2 IRES, Martineau et al., Mol. Cell. Biol., 24(17): 7622-7635 (2004)), vascular endothelial growth factor IRES (Baranick et al., Proc. Natl. Acad. Sci. U.S.A., 105(12): 4733-4738 (2008); Stein et al., Mol. Cell. Biol., 18(6): 3112-3119 (1998); Bert et al., RNA, 12(6): 1074-1083(2006)), and insulin-like growth factor 2 IRES (Pedersen et al., Biochem. J., 363(Pt 1): 37-44 (2002)).

In some cases, a recombinant circular RNA comprises a sequence encoding a protein or polypeptide operably linked to an IRES. A recombinant circular RNA comprising an IRES can be designed to produce any polypeptide of interest of appropriate size. For example, a circular RNA may comprise an IRES operably linked to an RNA sequence encoding an immunogenic polypeptide, such as an antigen from a bacterium, virus, fungus, protist, or parasite. Alternatively, a circular RNA may comprise an IRES operably linked to an RNA sequence encoding a therapeutic polypeptide such as an enzyme, hormone, neurotransmitter, cytokine, antibody, tumor suppressor, or cytotoxic agent for treating a genetic disorder, cancer, or other disease.

IRES elements are known in the art and nucleotide sequences and vectors encoding same are commercially available from a variety of sources, such as, for example, Clontech (Mountain View, CA), Invivogen (San Diego, CA), Addgene (Cambridge, MA) and GeneCopoeia (Rockville, MD), and IRESite: The database of experimentally verified IRES structures (iresite.org).

Polynucleotides encoding the desired RNAs, polypeptides, introns, and IRESs for use in the present disclosure can be made using standard molecular biology techniques. For example, polynucleotide sequences can be made using recombinant methods, such as by screening cDNA and genomic libraries from cells, or by excising the polynucleotides from a vector known to include same. Polynucleotides can also be produced synthetically, rather than cloned, based on the known sequences. The complete sequence can be assembled from overlapping oligonucleotides prepared by standard methods, then assembled and ligated into the complete sequence (see, e.g., Edge, Nature, 292: 756 (1981); Nambair et al., Science, 223: 1299 (1984); and Jay et al., J. Biol. Chem., 259: 6311(1984)). Other methods for obtaining or synthesizing nucleic acid sequences include, but are not limited to, site-directed mutagenesis and polymerase chain reaction (PCR) techniques (disclosed in, e.g., Greene, M.R., and Sambrook, J., Molecular Cloning: A Laboratory Manual, Cold Spring Harbor Laboratory Press; 4th edition (June 15, 2012)), an automated polynucleotide synthesizer (see, e.g., Jayaraman et al., Proc. Natl. Acad. Sci. USA, 88: 4084-4088 (1991)), oligonucleotide-directed synthesis (Jones et al., Nature, 54: 75-82 (1986)), oligonucleotide directed mutagenesis of preexisting nucleotide regions (Riechmann et al., Nature 332: 323-327 (1988); and Verhoeyen et al., Science, 239: 1534-1536 (1988)), and enzymatic filling-in of gapped oligonucleotides using T4 DNA polymerase (Queen et al., Proc. Natl. Acad. Sci. USA, 86: 10029-10033(1989)).

The recombinant circular RNA molecule may be of any suitable length or size. For example, the recombinant circular RNA molecule may comprise between about 200 nucleotides and about 6,000 nucleotides (e.g., about 300, 400, 500, 600, 700, 800, 900, 1,000, 2,000, 3,000, 4,000, 5,000 nucleotides, or a range defined by any two of the foregoing values). In some embodiments, the recombinant circular RNA molecule comprises between about 500 and about 3,000 nucleotides (about 550, 650, 750, 850, 950, 1,100, 1,200, 1,300, 1,400, 1,500, 1,600, 1,700, 1,800, 1,900, 2,100, 2,200, 2,300, 2,400, 2,500, 2,600, 2,700, 2,800, 2,900 nucleotides, or a range defined by any two of the foregoing values). In one embodiment, the recombinant circular RNA molecule comprises about 1,500 nucleotides.

### circRNA as an Adjuvant

circRNA molecules that do not contain m⁶A can be used to provoke an immune response in a subject. Thus, in some embodiments, a circRNA lacking m⁶A may be used as an adjuvant, for example as a part of a vaccine composition.

In some embodiments, an immunogenic circular RNA is administered to a subject in need thereof. In some embodiments, the immunogenic circular RNA does not contain any m⁶A residues.

In some embodiments, the circular RNA comprises a sequence encoding a polypeptide. The polypeptide may be, for example, an antigenic polypeptide. In some embodiments, the polypeptide comprises multiple (i.e., at least two) antigens. The antigen may be of viral, bacterial, parasitic, fungal, protozoan, prion, cellular, or extracellular origin. In some embodiments, the at least one antigen is a tumor antigen. In some embodiments, the circular RNA of the vaccine composition comprises an internal ribosome entry site (IRES) that is operably linked to the sequence encoding a polypeptide.

In some embodiments, the circular RNA is synthesized ex vivo before administration to the subject. In some embodiments, the circular RNA is produced using *in vitro* transcription.

In some embodiments, the circular RNA is administered to a subject as naked RNA. In some embodiments, the circular RNA is complexed with a nanoparticle such as, for example, a polyethylenimine (PEI) nanoparticle.

In some embodiments, a vector comprising a DNA sequence encoding the circular RNA is administered to the subject. In some embodiments, the DNA sequence encoding the circular RNA comprises features that prevent m⁶a modification of the circular RNA. For example, the DNA sequence may not comprise and RRACH motifs (SEQ ID NO: 17). The vector may be, for example, a non-viral vector such as a plasmid. In some embodiments, the vector is a viral vector, such as an adenovirus vector, an adeno-associated virus vector, a retrovirus vector, a lentivirus vector, or a herpesvirus vector.

In some embodiments, the vector is targeted to one or more specific cell types. For example, the vector may specifically or preferentially bind to one cell type, and not to another cell type. In some embodiments, the vector is targeted to a cancer cell.

In some embodiments, a vaccine composition comprises a circular RNA. In some embodiments, a vaccine composition comprises a circular RNA molecule that does not contain any N6-methyladenosine (m⁶A) residues. In some embodiments, the circular RNA lacks an RRACH motif (SEQ ID NO: 18). In some embodiments, the circular RNA comprises one or more RRUCH motifs (SEQ ID NO: 20).

In some embodiments, the vaccine composition comprises a circular RNA molecule that does not contain any N6-methyladenosine (m⁶A) residues, and also comprises at least one antigen.

In some embodiments, the circular RNA of the vaccine composition is produced using *in vitro* transcription. In some embodiments, the circular RNA is present in the composition as naked RNA. In some embodiments, the circular RNA is complexed with a nanoparticle such as, for example, a polyethylenimine (PEI) nanoparticle.

The vaccine composition may be administered to a subject in need thereof to treat or prevent a disease, disorder, or condition. Accordingly, in some embodiments, a method of eliciting an innate immune response in a subject in need thereof comprises administering to the subject an effective amount of the vaccine composition.

### circRNA as Delivery Vehicle

As N6-methyladenosine (m⁶A) modification of non-native circRNAs inhibits the innate immune response induced thereby, m⁶A-modified circRNA molecules can be used to deliver various substances to cells without being cleared by the host immune system. Thus, the present disclosure also provides a method of delivering a substance to a cell, which comprises: (a) generating a recombinant circular RNA molecule that comprises at least one N6-methyladenosine (m⁶A); (b) attaching a substance to the recombinant circular RNA molecule to produce a complex comprising the recombinant circular RNA molecule attached to the substance; and (c) contacting a cell with the complex, whereby the substance is delivered to the cells. Descriptions of the recombinant circular RNA molecule, m⁶A modification, methods of generating a recombinant circular RNA molecule, and components thereof as described above also apply to those same aspects of the method of delivering a substance to a cell.

Any suitable substance, compound, or material can be delivered to a cell using the disclosed circular RNA molecule. The substance may be a biological substance and/or a chemical substance. For example, the substance may be a biomolecule, such as a protein (e.g., a peptide, polypeptide, protein fragment, protein complex, fusion protein, recombinant protein, phosphoprotein, glycoprotein, or lipoprotein), lipid, nucleic acid, or carbohydrate. Other substances that may be delivered to a cell using the disclosed circular RNA molecule include, but are not limited to, hormones, antibodies, growth factors, cytokines, enzymes, receptors (e.g., neural, hormonal, nutrient, and cell surface receptors) or their ligands, cancer markers (e.g., PSA, TNF-alpha), markers of myocardial infarction (e.g., troponin or creatine kinase), toxins, drugs (e.g., drugs of addiction), and metabolic agents (e.g., including vitamins). In some embodiments, the substance is protein or peptide, such as an antigen, epitope, cytokine, toxin, tumor suppressor protein, growth factor, hormone, receptor, mitogen, immunoglobulin, neuropeptide, neurotransmitter, or enzyme. When the substance is an antigen or an epitope, the antigen or epitope can be obtained or derived from a pathogen (e.g., a virus or bacterium), or a cancer cell (i.e., a "cancer antigen" or "tumor antigen").

In other embodiments, the substance may be a small molecule. The term "small molecule," as used herein, refers to a low molecular weight (< 900 daltons) organic compound that may regulate a biological process, with a size typically on the order of 1 nm. Small molecules exhibit a variety of biological functions and may serve a variety applications, such as in cell signaling, as pharmaceuticals, and as pesticides. Examples of small molecules include amino acids, fatty acids, phenolic compounds, alkaloids, steroids, bilins, retinoids, etc.

Any suitable method for conjugation of biomolecules may be used to attach the substance to the recombinant circular RNA molecule to form a complex comprising the recombinant circular RNA molecule attached to the substance. Ideally, the substance is covalently linked to the recombinant circular RNA molecule. Covalent linkage may occur by way of a linking moiety present on either the circular RNA molecule or the substance. The linking moiety desirably contains a chemical bond that may allow for the release of the substance inside a particular cell. Suitable chemical bonds are well known in the art and include disulfide bonds, acid labile bonds, photolabile bonds, peptidase labile bonds, and esterase labile bonds. Typical covalent conjugation methods target side chains of specific amino acids such as cysteine and lysine. Cysteine and lysine side chains contain thiol and amino groups, respectively, which allow them to undergo modification with a wide variety of reagents (e.g., linking reagents). Bioconjugation methods are further described in, e.g., N. Stephanopoulos & M.B. Francis, Nature Chemical Biology, 7: 876-884 (2011); Jain et al., Pharm Res., 32(11): 3526-40 (2015); and Kalia et al., Curr. Org. Chem., 14(2): 138-147 (2010).

Following formation of a complex comprising the substance attached to the recombinant circular RNA molecule, the method comprises contacting a cell with the complex, whereby the substance is delivered to the cell. Any suitable prokaryotic or eukaryotic cell may be contacted with the complex. Examples of suitable prokaryotic cells include, but are not limited to, cells from the genera *Bacillus* (such as *Bacillus subtilis* and *Bacillus brevis), Escherichia* (such as *E*. *coli*), *Pseudomonas, Streptomyces, Salmonella,* and *Erwinia.* Particularly useful prokaryotic cells include the various strains of *Escherichia coli* (e.g., K12, HB101 (ATCC No. 33694), DH5α, DH10, MC1061 (ATCC No. 53338), and CC102).

Suitable eukaryotic cells are known in the art and include, for example, yeast cells, insect cells, and mammalian cells. Examples of suitable yeast cells include those from the genera *Hansenula, Kluyveromyces, Pichia, Rhinosporidium, Saccharomyces,* and *Schizosaccharomyces.* Suitable insect cells include Sf-9 and HIS cells (Invitrogen, Carlsbad, Calif.) and are described in, for example, Kitts et al., Biotechniques, 14: 810-817 (1993); Lucklow, Curr. Opin. Biotechnol., 4: 564-572 (1993); and Lucklow et al., J. Virol., 67: 4566-4579 (1993).

In certain embodiments, the cell is a mammalian cell. A number of suitable mammalian cells are known in the art, many of which are available from the American Type Culture Collection (ATCC, Manassas, Va.). Examples of suitable mammalian cells include, but are not limited to, Chinese hamster ovary cells (CHO) (ATCC No. CCL61), CHO DHFR-cells (Urlaub et al., Proc. Natl. Acad. Sci. USA, 97: 4216-4220 (1980)), human embryonic kidney (HEK) 293 or 293T cells (ATCC No. CRL1573), and 3T3 cells (ATCC No. CCL92). Other suitable mammalian cell lines are the monkey COS-1 (ATCC No. CRL1650) and COS-7 cell lines (ATCC No. CRL1651), as well as the CV-1 cell line (ATCC No. CCL70). Further exemplary mammalian host cells include primate cell lines and rodent cell lines, including transformed cell lines. Normal diploid cells, cell strains derived from *in vitro* culture of primary tissue, as well as primary explants also are suitable. Other suitable mammalian cell lines include, but are not limited to, mouse neuroblastoma N2A cells, HeLa, mouse L-929 cells, and BHK or HaK hamster cell lines, all of which are available from the ATCC. Methods for selecting suitable mammalian host cells and methods for transformation, culture, amplification, screening, and purification of such cells are well known in the art (see, e.g., Ausubel et al., eds., Short Protocols in Molecular Biology, 5th ed., John Wiley & Sons, Inc., Hoboken, N.J. (2002)).

Preferably, the mammalian cell is a human cell. For example, the mammalian cell can be a human immune cell, particularly a cell that can present an antigen or epitope to the immune system. Examples of human immune cells include lymphocytes (e.g., B or T lymphocytes), monocytes, macrophages, neutrophils, and dendritic cells. In one embodiment, the cell is a macrophage.

The complex comprising the recombinant circular RNA molecule attached to the substance may be introduced into a cell by any suitable method, including, for example, by transfection, transformation, or transduction. The terms "transfection," "transformation," and "transduction" are used interchangeably herein and refer to the introduction of one or more exogenous polynucleotides into a host cell by using physical or chemical methods. Many transfection techniques are known in the art and include, for example, calcium phosphate DNA co-precipitation; DEAE-dextran; electroporation; cationic liposome-mediated transfection; tungsten particle-facilitated microparticle bombardment; and strontium phosphate DNA co-precipitation.

In some embodiments, the complex may be delivered to a cell in the form of naked RNA conjugated to the substance. In some embodiments, the complex may be complexed with a nanoparticle for delivery to the cell, such as a polyethylenimine (PEI) nanoparticle.

In some embodiments, a composition comprises the RNA conjugated to the substance and may optionally comprise a pharmaceutically acceptable carrier. The choice of carrier will be determined in part by the particular circular RNA molecule and type of cell (or cells) into which the circular RNA molecule is introduced. Accordingly, a variety of suitable formulations of the composition are possible. For example, the composition may contain preservatives, such as, for example, methylparaben, propylparaben, sodium benzoate, and benzalkonium chloride. A mixture of two or more preservatives optionally may be used. In addition, buffering agents may be used in the composition. Suitable buffering agents include, for example, citric acid, sodium citrate, phosphoric acid, potassium phosphate, and various other acids and salts. A mixture of two or more buffering agents optionally may be used. Methods for preparing compositions for pharmaceutical use are known to those skilled in the art and are described in more detail in, for example, Remington: The Science and Practice of Pharmacy, Lippincott Williams & Wilkins; 21st ed. (May 1, 2005).

In other embodiments, the composition containing the complex comprising the recombinant circular RNA molecule attached to the substance can be formulated as an inclusion complex, such as cyclodextrin inclusion complex, or as a liposome. Liposomes can be used to target host cells or to increase the half-life of the circular RNA molecule. Methods for preparing liposome delivery systems are described in, for example, Szoka et al., Ann. Rev. Biophys. Bioeng., 9: 467 (1980), and U.S. Patents 4,235,871; 4,501,728; 4,837,028; and 5,019,369. The complex may also be formulated as a nanoparticle.

### circRNA for Sequestering RNA Binding Proteins

The disclosure also provides a method of sequestering an RNA-binding protein in a cell, which comprises (a) generating a recombinant circular RNA molecule that comprises at least one N6-methyladenosine (m⁶A) and one or more RNA-binding protein binding motifs; and (b) contacting a cell comprising the RNA-binding protein with the recombinant circular RNA molecule, whereby the RNA-binding protein binds to the one more RNA-binding protein binding motifs and is sequestered in the cell. Descriptions of the recombinant circular RNA molecule, m⁶A modification, methods of generating a recombinant circular RNA molecule, methods of contacting a cell with circRNA, and components thereof as described above also apply to those same aspects of the method of sequestering an RNA-binding protein in a cell.

RNA-binding proteins play primary roles in RNA metabolism, coordinating networks of RNA-protein and protein-protein interactions, and regulating RNA splicing, maturation, translation, transport, and turnover. Aberrant expression, dysfunction, and aggregation of RNA-binding proteins have been identified in several major classes of human diseases, including neurological disorders, muscular atrophies, and cancer. Thus, the RNA-binding protein, particularly when aberrantly expressed in a cell, may be associated with a disease.

RNA-binding proteins typically contain one or more RNA recognition motifs (RRMs) (also referred to as "RNA-binding motifs^{"}). Numerous RRMs are known for a variety of different RNA-binding proteins. The ribonucleoprotein (RNP) domain (also known as the "RNA recognition motif (RRM)" and "RNA-binding domain (RBD)") is one of the most abundant protein domains in eukaryotes. The RNP domain contains an RNA-binding domain of approximately 90 amino acids which includes two consensus sequences: RNP-1 and RNP-2. RNP-1 comprises eight conserved residues that are mainly aromatic and positively charged, while RNP-2 is a less conserved sequence comprised of six amino acid residues. The RNP domain has been shown to be necessary and sufficient for binding RNA molecules with a wide range of specificities and affinities. Other RNA-binding domains include, but are not limited to, zinc finger domains, hnRNP K homology (KH) domains, and double-stranded RNA binding motifs (dsRBMs) (see, e.g., Cléry A, H.-T. Allain F., From Structure to Function of RNA Binding Domains. In: Madame Curie Bioscience Database, Austin (TX): Landes Bioscience (2000-2013)). The recombinant circular RNA molecule is generated to contain one or more domains recognized by the RRMs or RNA-binding motifs (i.e., "RNA-binding protein binding domains"). The choice of RNA-binding protein binding domain to include in the recombinant circRNA molecule will depend upon the specific RNA binding protein targeted for sequestration in a cell. A recombinant circular RNA molecule can be generated to include one or more RNA-binding protein binding domains using routine molecular biology and/or recombinant DNA techniques.

In certain embodiments, the RNA-binding protein is aberrantly expressed in the cell that is contacted with the recombinant circRNA molecule. As mentioned above, aberrant expression of RNA-binding proteins has been associated with diseases such as neurological disorders, muscular atrophies, and cancer. Expression of the RNA-binding protein is "aberrant" in that it is abnormal. In this regard, the gene encoding the RNA-binding protein may be abnormally expressed in the cell, resulting in abnormal amounts of the RNA-binding protein. Alternatively, gene expression may be normal, but production of the RNA-protein is dysregulated or dysfunctional so as to result in abnormal amounts of the protein in the cell. Aberrant expression includes, but is not limited to, overexpression, underexpression, complete lack of expression, or temporal dysregulation of expression (e.g., a gene expressed at inappropriate times in a cell). Expression of a mutant or variant RNA-binding protein at normal levels in a cell may also be considered aberrant expression of the RNA-binding protein. Thus, in some embodiments, the RNA-binding protein is encoded by a nucleic acid sequence comprising at least one mutation (e.g., a deletion, insertion, or substitution).

In some embodiments, the circular RNA may be to a cell in the form of naked RNA. In some embodiments, the circular RNA may be complexed with a nanoparticle for delivery to the cell, such as a polyethylenimine (PEI) nanoparticle.

### Modulation of circRNA Innate Immunogenicity

It may be desirable to modulate the innate immunogenicity of a circular RNA molecule, depending on the ultimate application thereof. The terms "innate immunogenicity" and "innate immunity" are used interchangeably herein and refer to the nonspecific defense mechanisms that arise immediately or within hours of exposure to an antigen. These mechanisms include physical barriers such as skin, chemicals in the blood, and immune system cells that attack foreign cells in an organism. For example, when the circRNA molecule is used to sequester RNA-binding proteins in a cell, the innate immunogenicity induced by the circRNA molecule may be reduced so as to reduce clearance thereof and maximize the efficacy of protein sequestration. In this regard, the disclosure provides a method of reducing the innate immunogenicity of a circular RNA molecule in a subject, wherein the method comprises: (a) providing a circular RNA molecule that induces an innate immune response in a subject; and (b) introducing at least one nucleoside selected from N6-methyladenosine (m⁶A), pseudouridine, and inosine into the circular RNA molecule to provide a modified circular RNA molecule having reduced innate immunogenicity. Descriptions of circular RNA molecules, m⁶A modification, methods of generating a recombinant circular RNA molecule, and components thereof as described above also apply to those same aspects of the method of reducing the innate immunogenicity of a circular RNA in a subject.

Pseudouridine (also referred to as "psi" or "> "), one of the most abundant modified nucleosides found in RNA, is present in a wide range of cellular RNAs and is highly conserved across species. Pseudouridine is derived from uridine (U) via base-specific isomerization catalyzed by Ψ sythnases. Inosine is a nucleoside that is formed when hypoxanthine is attached to a ribose ring (also known as a ribofuranose) via a β-N9-glycosidic bond. Inosine is commonly found in tRNAs and is essential for proper translation of the genetic code in wobble base pairs. Figure 15 demonstrates that introduction of inosine or pseudouridine into circular RNA impacts circRNA immunity. Without being bound by any theory, it is believed that introduction of inosine or pseudouridine into the circular RNA prevents m⁶A modification thereof. Ideally, at least 1% (e.g., 1%, 2%, 3%, 4%, 5%, 6%, 7%, 8%, 9% or more) of the of the circular RNA molecule contains m⁶A, pseudouridine, and/or inosine. In other embodiments, at least 10% (e.g., 10%, 11%, 12%, 13%, 14%, 15%, 20%, 30%, 40%, 50%, 60%, 70%, 80%, 90%, or more) of the circular RNA molecule contains m⁶A, pseudouridine, and/or inosine.

Alternatively, in embodiments where the circRNA is used to deliver an antigenic protein (e.g., a tumor or cancer antigen) to cells, the innate immunogenicity of the circRNA molecule may be increased. To this end, the disclosure also provides a method of increasing the innate immunogenicity of a circular RNA molecule in a subject, which method comprises: (a) generating a circular RNA molecule which lacks an RRACH motif (SEQ ID NO: 18); and/or (b) replacing one or more adenosines in the at least one exon with another base (e.g., U, G, C, or inosine) to provide a modified circular RNA molecule having increased innate immunogenicity. Descriptions of circular RNA molecules, methods of generating a recombinant circular RNA molecule, and components thereof as described above also apply to those same aspects of the method of increasing the innate immunogenicity of a circular RNA in a subject.

As discussed in the Examples below, RRACH (SEQ ID NO: 17-18) is a consensus motif for m⁶A modification. Thus, in some embodiments, a circular RNA molecule may be engineered to lack an RRACH motif (SEQ ID NO: 18) by replacing the "A" in the motif with another base or combination of bases, such as a uracil ("U"), guanine ("G"), or cytosine ("C"); however any nucleotide in a RRACH motif may be replaced with another base or combination of bases. Ideally, at least 1% (e.g., 1%, 2%, 3%, 4%, 5%, 6%, 7%, 8%, 9% or more) of the adenosines in the circular RNA molecule are replaced with another base (e.g., uracils) or combinations of bases. In other embodiments, at least 10% (e.g., 10%, 11%, 12%, 13%, 14%, 15%, 20%, 30%, 40%, 50%, 60%, 70%, 80%, 90%, or more) of the adenosines in the circular RNA molecule are replaced with another base (e.g., uracils) or combination of bases. For example, all (i.e., 100%) of the adenosines in the circular RNA molecule may be replaced with another base (e.g., uracils) or combination of bases.

The method of reducing or increasing the innate immunogenicity of a circular RNA molecule may further comprise administering the modified circular RNA to a subject. The modified circular RNA, or a composition comprising same, can be administered to a subject (e.g., a mammal) using standard administration techniques, including oral, intravenous, intraperitoneal, subcutaneous, pulmonary, transdermal, intramuscular, intranasal, buccal, sublingual, vaginal, or suppository administration.

In some embodiments, the circular RNA may be delivered to a cell in the form of naked RNA. In some embodiments, the circular RNA may be complexed with a nanoparticle for delivery to the cell, such as a polyethylenimine (PEI) nanoparticle.

The following examples further illustrate the invention but, of course, should not be construed as in any way limiting its scope.

### Embodiments

1. A vaccine composition comprising a circular RNA molecule that does not contain any N6-methyladenosine (m⁶A) residues.
2. The vaccine composition of embodiment 1, wherein the circular RNA lacks an RRACH motif.
3. The vaccine composition of any one of embodiments 1-2, wherein the vaccine composition further comprises at least one antigen.
4. The vaccine composition of any one of embodiments 1-2, wherein the circular RNA molecule comprises an internal ribosome entry site (IRES) that is operably linked to a sequence encoding a polypeptide.
5. The vaccine composition of embodiment 4, wherein the sequence encoding a polypeptide encodes at least one antigen.
6. The vaccine composition of any one of embodiments 3 or 5, wherein the at least one antigen is of viral, bacterial, parasitic, fungal, protozoan, prion, cellular, or extracellular origin.
7. The vaccine composition of any one of embodiments 3 or 5, wherein the at least one antigen is a tumor antigen.
8. The vaccine composition of any one of embodiments 1-7, wherein the circular RNA molecule is produced using *in vitro* transcription.
9. The vaccine composition of any one of embodiments 1-8, wherein the circular RNA is present in the composition as naked RNA.
10. The vaccine composition of any one of embodiments 1-8, wherein the circular RNA is complexed with a nanoparticle.
11. The vaccine composition of embodiment 10, wherein the nanoparticle is a polyethylenimine (PEI) nanoparticle.
12. A method of eliciting an innate immune response in a subject in need thereof, the method comprising administering to the subject an effective amount of the vaccine composition of any one of embodiments 1-11.
13. A composition comprising a DNA sequence coding a circular RNA, wherein the circular RNA does not contain any N6-methyladenosine (m⁶A) residues.
14. The composition of embodiment 13, wherein the DNA sequence does not comprise any RRACH motifs.
15. The composition of embodiment 13 or 14, wherein a viral or a non-viral vector comprises the DNA sequence.
16. The composition of embodiment 15, wherein the viral vector is an adenovirus vector, an adeno-associated virus vector, a retrovirus vector, a lentivirus vector, or a herpesvirus vector.
17. The composition of embodiment 15, wherein the non-viral vector is a plasmid.
18. A method of eliciting an innate immune response in a subject in need thereof, the method comprising administering to the subject an effective amount of the composition of any one of embodiments 13-17.
19. A method of producing a circular RNA molecule by *in vitro* transcription, the method comprising:
   (a) providing a DNA template encoding the circular RNA molecule, ribonucleotide triphosphates, and a RNA polymerase;
   (c) transcribing a linear RNA from the DNA template; and
   (d) circularizing the linear DNA to form a circular RNA;
      wherein the ribonucleotide triphosphates do not include any N6-methyladenosine-5'-triphosphate (m⁶ATP); and
      wherein the circular RNA is capable of producing an innate immune response in the subject.
20. The method of embodiment 19, wherein the circular RNA does not comprise any m⁶A.
21. A method of producing a circular RNA molecule by *in vitro* transcription, the method comprising:
   (a) providing a DNA template encoding the circular RNA molecule, ribonucleotide triphosphates, and a RNA polymerase;
   (c) transcribing a linear RNA from the DNA template; and
   (d) circularizing the linear DNA to form a circular RNA;
      wherein the ribonucleotide triphosphates comprise N6-methyladenosine-5'-triphosphate (m⁶ATP); and
      wherein the circular RNA is less immunogenic compared to a circular RNA produced using the same method but in the absence of m⁶ATP.
22. The method of embodiment 21, wherein at least 1% of the adenosines in the recombinant circular RNA molecule are N6-methyladenosine (m⁶A).
23. The method of embodiment 22, wherein at least 10% of the adenosines in the recombinant circular RNA molecule are N6-methyladenosine (m⁶A).
24. The method of embodiment 23, wherein all of the adenosines in the recombinant circular RNA molecule are N6-methyladenosine (m⁶A).
25. A method of reducing the innate immunogenicity of a circular RNA molecule, wherein the method comprises:
   (a) providing a circular RNA molecule that induces an innate immune response in a subject; and
   (b) introducing at least one nucleoside selected from N6-methyladenosine (m⁶A), pseudouridine, and inosine into the circular RNA molecule to provide a modified circular RNA molecule having reduced innate immunogenicity.
26. The method of embodiment 25, wherein the method further comprises administering the modified circular RNA to a subject.
27. The method of embodiment 25 or 26, wherein at least 1% of the of the circular RNA molecule contains m⁶A, pseudouridine, and/or inosine.
28. The method of embodiment 21, wherein at least 10% of the circular RNA molecule contains m⁶A, pseudouridine, and/or inosine.
29. A method of increasing the innate immunogenicity of a circular RNA molecule, wherein the method comprises:
   (a) generating a circular RNA molecule which lacks an RRACH motif; and
   (b) replacing one or more adenosines with another base to provide a modified circular RNA molecule having increased innate immunogenicity.
30. The method of embodiment 29, wherein the method further comprises administering the modified circular RNA to a subject.
31. The method of embodiment 29 or 30, wherein at least 1% of the adenosines in the circular RNA molecule are replaced with uracils.
32. The method of embodiment 30, wherein at least 10% of the adenosines in the circular RNA molecule are replaced with uracils.
33. The method of embodiment 32, wherein all of the adenosines in the circular RNA molecule are replaced with uracils.
34. A method of delivering a substance to a cell, wherein the method comprises:
   (a) generating a recombinant circular RNA molecule that comprises at least one N6-methyladenosine (m⁶A);
   (b) attaching a substance to the recombinant circular RNA molecule to produce a complex comprising the recombinant circular RNA molecule attached to the substance; and
   (c) contacting a cell with the complex, whereby the substance is delivered to the cell.
35. The method of embodiment 34, wherein the substance is a protein or peptide.
36. The method of embodiment 34 or 35, wherein the substance is an antigen or an epitope.
37. The method of embodiment 34, wherein the substance is a small molecule.
38. The method of any one of embodiments 34-37, wherein the substance is covalently linked to the recombinant circular RNA molecule.
39. A method of sequestering an RNA-binding protein in a cell, wherein the method comprises:
   (a) generating a recombinant circular RNA molecule that comprises at least one N6-methyladenosine (m⁶A) and one or more RNA-binding protein binding domains; and
   (b) contacting a cell comprising the RNA-binding protein with the recombinant circular RNA molecule, whereby the RNA-binding protein binds to the one more RNA-binding protein binding domains and is sequestered in the cell.
40. The method of embodiment 39, wherein RNA-binding protein is aberrantly expressed in the cell.
41. The method of embodiment 39 or 40, wherein the RNA-binding protein is encoded by a nucleic acid sequence comprising at least one mutation.
42. The method of any one of embodiments 39-41, wherein the RNA-binding protein is associated with a disease.
43. The method of any one of embodiments 39-42, wherein at least 1% of the adenosines in the recombinant circular RNA molecule are N6-methyladenosine (m⁶A).
44. The method of embodiment 43, wherein at least 10% of the adenosines in the recombinant circular RNA molecule are N6-methyladenosine (m⁶A).
45. The method of embodiment 44, wherein all of the adenosines in the recombinant circular RNA molecule are N6-methyladenosine (m⁶A).
46. The method of any one of embodiments 39-45, wherein the recombinant RNA molecule comprises a self-splicing group I intron of the phage T4 thymidylate synthase (td) gene and at least one exon.
47. The method of any one of embodiments 39-46, wherein the recombinant circular RNA molecule comprises an internal ribosome entry site (IRES).
48. The method of any one of embodiments 39-47, wherein the recombinant circular RNA molecule comprises between 200 nucleotides and 6,000 nucleotides.
49. The method of embodiment 48, wherein the recombinant circular RNA molecule comprises about 1,500 nucleotides.

### EXAMPLES

The following materials and methods were used in the experiments described in the Examples.

### Plasmids

Plasmids encoding phage introns that express circRNA through autocatalytic splicing were previously described in (Chen et al., *supra).* IN-FUSION^{®} HD assembly (Takara Bio, 638910) was used to construct the plasmid encoding phage introns expressing foreign circGFP with a BoxB motif incorporated. Plasmids expressing YTHDF1N and YTHDF2N with and without λN were provided by Dr. Chuan He (University of Chicago). Plasmids expressing YTHDF2 protein domain truncations were constructed with IN-FUSION^{®} HD. All plasmids were propagated in NEB^{®} Turbo Competent *E*. *coli* cells (New England Biolabs, C2984H) grown in LB medium and purified using the ZYMOPURE II^{™} Plasmid Prep Kits (Zymo Research, D4200).

### RNA Synthesis and Purification

RNA was synthesized by *in vitro* transcription using MEGAscript T7 transcription kit (Ambion, AM1334) following the manufacturer's instructions and incubation at 37 °C overnight, or for at least 8 hours. m⁶A-labeled RNA was synthesized in the same way by *in vitro* transcription using MEGASCRIPT^{®} T7 transcription kit (Ambion, AM1334) and adding m⁶ATP (Trilink, N-1013) in the specified ratio with the transcription kit's ATP. Transcribed circFOREIGN was purified by RNEASY^{®} Mini column (Qiagen, 74106), then treated with RNase R (Epicenter, RNR07250) in the following manner: circFOREIGN secondary structure was denatured at 72 °C for five minutes followed by two minutes on ice; RNaseR was added at a ratio of 1U:1 > g of RNA and incubated at 37 °C for 2-3 hours. CircRNALinear RNA was not treated with RNase R. CircFOREIGN was then purified by RNEASY^{®} column. CircFOREIGN or linear RNA were then phosphatase treated by FASTAP^{™} in the following manner: FASTAP^{™} was added at a ratio of 1U: 1> > g of circFOREIGN, incubated at 37 °C for 2 hours, then purified by RNEASY^{®} column. RNA quality was assessed by Tapestation analysis (Agilent, 5067-5576).

CircFOREIGN was gel purified by denaturing RNA with Gel Loading Buffer II (Thermo Fisher Scientific, AM8547) at 72 °C for three minutes followed by two minutes on ice, then loaded on 1% low melting point agarose. Gel extraction was done on a blue light transilluminator (Clare Chemical) followed by ZYMOCLEAN^{™} Gel Recovery Kit (Zymo Research, R1011) purification following the manufacturer's instructions except for melting, which was done rotating at room temperature for 10 minutes.

HPLC fractionation was performed with a 4.6×300mm size exclusion column (Sepax Technologies, 215980P-4630) with particle size of 5 > m and pore size of 2000 Å. Nuclease-free TE buffer was used as the mobile phase at a flow rate of 0.3 ml/minute. RNA fractions were manually collected, lyophilized, and then cleaned with RNA Clean & Concentrator-5 (Zymo Research, R1013) prior to subsequent quality control and experimental use.

### m⁶A-irCLIP

10 µg of total RNA was enriched for circRNA by removing mRNAs (polyA-) using the Poly(A)Purist MAG Kit (Thermo Fisher Scientific, AM1922) and removing ribosomal RNAs (ribo-) using the RIBOMINUS^{™} Eukaryote System v2 kit (Thermo Fisher Scientific, A15026). The resulting polyA-/ribo- RNA was then fragmented to 35-100 nt sizes using the RNA Fragmentation Buffer (RNA) at 75 °C for 12 minutes. Fragmented RNA was denatured and then incubated with anti-m⁶A antibody (Synaptic Systems, 202003) for two hours at 4 °C in IPP buffer (50 mM Tris-HCl, pH 7.4; 100 mM NaCl; 0.05% NP-40; 5 mM EDTA). The RNA and antibody were then crosslinked using UV light (254 nm) using two rounds of crosslinking at 0.15J (Stratalinker 2400). The crosslinked RNA and antibody were then incubated with Protein A Dynabeads (Thermo Fisher Scientific, 10002D) for two hours at 4 °C. The beads were then washed with once with IPP buffer for 10 minutes at 4 °C with rotation, once with low salt buffer (50 mM Tris, pH 7.4; 50 mM NaCl; 1 mM EDTA; 0.1% NP-40) for 10 minutes at 4 °C with rotation, once with high salt buffer (50 mM Tris-HCl pH 7.4, 1M NaCl, 1% NP-40, 0.1% SDS) for 10 minutes at 4 °C with rotation, transferred to a new 1.5 mL tube, and washed twice with PNK buffer (20 mM Tris-HCl, pH 7.4; 10 mM MgCl2; 0.2% Tween 20). Libraries were then prepared using the irCLIP method (Zarnegar et al., 2016). Libraries were checked for quality by Bioanalyzer and submitted for sequencing on NextSeq 500 with custom sequencing primer P6_seq, as described in the irCLIP method. Reads were mapped to the hg38 and subsequently to a custom assembly of the circGFP sequence and PCR duplicates were removed using UMI-tools (Smith et al., 2017). Reproducible RT stops were identified using the FAST-iCLIP pipeline (Flynn et al., 2015).

### m⁶A-RIP-seq

10 µg of total RNA was enriched for circRNA by removing mRNAs (polyA-) using the Poly(A)Purist MAG Kit (Thermo Fisher Scientific, AM1922) and removing ribosomal RNAs (ribo-) using the RIBOMINUS^{™} Eukaryote System v2 kit (Thermo Fisher Scientific, A15026). The remaining RNA was then treated with RNase R to remove residual linear RNAs. The polyA-/ribo- RNase R+ RNA was then fragmented for 12 minutes at 75 °C with RNA Fragmentation Buffer (Thermo Fisher Scientific, AM8740). 3 µg anti-m⁶A (Synaptic Systems, 202003) was bound to Protein A Dynabeads for 2 hours at room temperature. Antibody bound beads were then washed with IPP buffer (50mM Tris-HCl, pH 7.4; 100mM NaCl; 0.05% NP-40; 5mM EDTA) and resuspend in IPP with 1 µL RIBOLOCK^{™} (Thermo Fisher Scientific, EO0382). Fragmented RNA in IPP buffer was incubated with antibody and beads for two hours at 4 °C with rotation. RNA bound beads were then washed once with IPP buffer for 10 minutes at 4 °C with rotation, once with low-salt buffer (50 mM Tris, pH 7.4; 50 mM NaCl; 1 mM EDTA; 0.1% NP-40) for 5 minutes at 4 °C with rotation, once with high-salt buffer (50 mM Tris-HCl pH 7.4, 1M NaCl, 1% NP-40, 0.1% SDS) for 5 minutes at 4 °C with rotation. Beads were then resuspended in 300 µL high-salt buffer and transfer to a new 1.5 mL tube. Beads were washed with PNK buffer (20 mM Tris-HCl, pH 7.4; 10 mM MgCl2; 0.2% Tween 20) and then resuspended in 500 µL Trizol and incubated for 5 minutes at 25 °C. 150 µL chloroform: isoamyl alcohol was added and mixed before incubating at 25 °C for 2 minutes. After spinning at 13,000xg at 4 °C for 10 minutes, the aqueous layer was transferred to a new 1.5 mL tube and cleaned up with RNA Clean & Concentrator-5 (Zymo Research, R1013). RNA was eluted in 10 µL nuclease-free water. To eluted RNA and 10% input RNA, 10 µL of end-repair mix was added (4 µL 5X PNK buffer; 1 µL RIBOLOCK^{™}, 1 µL FASTAP^{™}; 2 µL T4 PNK, 2 µL nuclease-free water). The reaction was incubated at 37 °C for one hour. 20 µL of linker ligation mix (2 µL 10X RNA ligation buffer; 2 µL 100 mM DTT; 2µL L3 linker (Zarnegar et al., 2016); 2 µL T4 RNA ligase buffer; 12 µL PEG8000 50% w/v) was added. The reaction was incubated for three hours at 25 °C and then cleaned up with an RNA Clean & Concentrator-5 column. Processed RNA was eluted in 10 µL of nuclease-free water. Libraries were prepared using the irCLIP method (Zarnegar et al., 2016) and sequenced on a NextSeq 500 using a custom sequencing primer (P6_seq (Zarnegar et al., 2016)). Reads were aligned to hg38 and circGFP sequence. Bam files were normalized to genome mapped reads.

### Reverse Transcription and Real Time PCR analysis (RT-qPCR)

Total RNA was isolated from cells using TRIZOL^{®} (Invitrogen, 15596018) and DIRECT-ZOL^{®} RNA Miniprep (Zymo Research, R2052) with on-column DNase I digestion following the manufacturers instructions. RT-qPCR analysis was performed in triplicate using Brilliant II SYBR Green qRT-PCR Master Mix (Agilent, 600825) and a LightCycler 480 (Roche). The primers used are shown in Table 1. mRNA levels were normalized to actin or GAPDH values. Relative expression of indicated mRNA genes for circRNA transfection were normalized by level of transfected RNA and plotted as the fold change to the expression level of cells with mock or linear RNA transfection.

**Table 1. qRT-PCR primers**

| **Oligo Name** | **Sequence** | **SEQ ID NO:** |
|---|---|---|
| hACTB1 qRT-PCR F | GAGGCACTCTTCCAGCCTT | 1 |
| hACTB1 qRT-PCR R | AAGGTAGTTTCGTGGATGCC | 2 |
| hRIG-I qRT-PCR F | TGTGGGCAATGTCATCAAAA | 3 |
| hRIG-I qRT-PCR R | GAAGCACTTGCTACCTCTTGC | 4 |
| hMDA5 qRT-PCR F | GGCACCATGGGAAGTGATT | 5 |
| hMDA5 qRT-PCR R | ATTTGGTAAGGCCTGAGCTG | 6 |
| hOAS1 qRT-PCR F | GCTCCTACCCTGTGTGTGTGT | 7 |
| hOAS1 qRT-PCR R | TGGTGAGAGTACTGAGGAAGA | 8 |
| hOASL qRT-PCR F | AGGGTACAGATGGGACATCG | 9 |
| hOASL qRT-PCR R | AAGGGTTCACGATGAGGTTG | 10 |
| hPKR qRT-PCR F | TCTTCATGTATGTGACACTGC | 11 |
| hPKR qRT-PCR R | CACACAGTCAAGGTCCTT | 12 |
| circRNA-junction qRT-PCR F | GATAAGCTTGCCACCTCAGTAGATG | 13 |
| circRNA-junction qRT-PCRR | ATCCATCACACTGGCATATGAC | 14 |
| linRNA qRT-PCR F | ACTACCTGAGCACCCAGTCC | 15 |
| linRNA qRT-PCR R | CTTGTACAGCTCGTCCATGC | 16 |

### Cell Lines and Maintenance

Human HeLa (cervical adenocarcinoma, ATCC CCL-2) and HEK293T (embryonic kidney, ATCC CRL-3216) cells were grown in Dulbecco's modified Eagle's medium (DMEM, Invitrogen, 11995-073) supplemented with 100 units/ml penicillin-streptomycin (Gibco, 15140-163) and 10% (v/v) fetal bovine serum (Invitrogen, 12676-011). Cell growth was maintained at 37 °C in a 5% CO₂ atmosphere.

### Cell Culture and Transient Transfection

Cells were plated 24 hours prior to transfection. Cells were at 70 to 80% confluence and transfected with RNA using Lipofectamine 3000 (Thermo Fisher Scientific, L3000008). 500 ng of linear RNA or circFOREIGN was transfected into one well of a 24-well plate using Lipofectamine 3000 (Thermo Fisher Scientific, L3000008). The nucleic acids with P3000 and Lipofectamine 3000 were diluted in Opti-MEM (Invitrogen, 31985-088) per manufacturer's instructions, and incubated for five minutes at room temperature. The nucleic acids and Lipofectamine 3000 were then mixed together, incubated for 15 minutes at room temperature, and then the nucleic acids-Lipofectamine 3000 complexes were applied dropwise to the monolayer cultures. In cases of ectopic protein expression, cells were electroporated with NEON^{™} Transfection System (Thermo Fisher Scientific MPK5000S) per the manufacturer's instructions. In most cases, cells were resuspended in buffer R at 2 x 10⁷/mL and 5 µg of DNA plasmid was electroporated with a 100 µL NEON^{™} tip. 12 hours later, cells were passaged and plated such that 24 hours later they would be 70 to 80% confluent. 24 hours later, cells were then transfected with RNA with Lipofectamine as described above. 24 hours after transfection, cells were washed once with PBS, and 300 µL of TRIZOL^{®} reagent was added per 24-well. RNA was harvested with DIRECT-ZOL^{®} RNA Miniprep.

### Western Blot Analysis

HeLa cells were collected and lysed 24 hours after transfection to extract total proteins. RIPA buffer (150 mM NaCl, 1% Triton X-100, 0.5% sodium deoxycholate, 0.1% SDS, 50 mM Tris, pH 8.0) was used to lyse the cells. Proteins were fractionated by sodium dodecyl sulfate polyacrylamide gel electrophoresis (SDS-PAGE), transferred to nitrocellulose membranes, blocked in phosphate-buffer saline containing 5% (wt/vol) nonfat milk for one hour at room temperature, and then incubated overnight at 4 °C with the primary antibody indicated in Table 2. IRDye 800CW Goat anti-rabbit IgG (Li-Cor, 926-32211) or IRDye 680CW Donkey anti-goat IgG (Li-Cor, 926-68074) secondary antibodies were used according to the manufacturer's instructions. Western blot detection and quantification was done using an Odyssey infrared imaging system (Li-Cor).

**Table 2**

| **REAGENT or RESOURCE** | **SOURCE** | **IDENTIFIER** |
|---|---|---|
| **Antibodies** | | |
| β-Actin (8H10D10) Mouse mAb (1:9000 for WB) | Cell Signaling Technology | 3700S |
| | | RRID: AB_2242334 |
| Anti-beta Actin antibody (1:1000 for WB) | Abcam | ab8227 |
| | | RRID: AB_230518: |
| Monoclonal ANTI-FLAG^{®} M2 antibody produced in mouse (1:1000 for WB) | Sigma-Aldrich | F1804 |
| | | RRID: AB_262044 |
| Anti-YTHDF1 antibody (1:1000 for WB) | Abcam | ab157542 |
| | | RRID: N/A |
| YTHDF2 Polyclonal Antibody (1:250 for WB) | Thermo Fisher Scientific | PA5-63756 |
| | | RRID: AB_2649742 |
| Recombinant Anti-METTL3 antibody [EPR18810] (1:1500 for WB) | Abcam | ab195352 |
| | | RRID: AB_2721254 |
| Rig-I (D14G6) Rabbit mAb (1:200 for IF) | Cell Signaling Technology | 3743S |
| | | RRID: AB_2269233 |
| Ub-K63 Monoclonal Antibody (HWA4C4) (1:200 for IF) | Thermo Fisher Scientific | 14-6077-82 |
| | | RRID: AB_1257213 |
| Mouse Anti-YTHDF2 polyclonal antibody (1:200 for IF) | USBiological Life Sciences | 135486 |
| | | RRID: N/A |
| IRDye^{®} 800CW Goat anti-Mouse IgG Secondary Antibody (1:15,000 for WB) | Li-COR Biosciences | 926-32210 |
| | | RRID: AB_621842 |
| IRDye^{®} 800CW Goat anti-Rabbit IgG Secondary Antibody (1:15,000 for WB) | Li-COR Biosciences | 926-32211 |
| | | RRID: AB_621843 |
| IRDye^{®} 680RD Goat anti-Mouse IgG Secondary Antibody (1:15,000 for WB) | Li-COR Biosciences | 926-68070 |
| | | RRID: AB_10956588 |
| IRDye^{®} 680RD Goat anti-Rabbit IgG Secondary Antibody (1:15,000 for WB) | Li-COR Biosciences | 926-68071 |
| | | RRID: AB_10956166 |
| Goat anti-Rabbit IgG (H+L) Highly Cross-Adsorbed Secondary Antibody, Alexa Fluor Plus 594 (1:2000 for IF) | Thermo Fisher Scientific | A32740 |
| | | RRID: AB_2762824 |
| Goat anti-Mouse IgG (H+L) Highly Cross-Adsorbed Secondary Antibody, Alexa Fluor Plus 647 (1:2000 for IF) | Thermo Fisher Scientific | A32728 |
| | | RRID: AB_2633277 |
| m6A Polyclonal rabbit purified antibody | Synaptic Systems | 202 003 |
| | | RRID: AB_2279214 |
| Anti-FLAG^{®} M2 Magnetic Beads | Sigma-Aldrich | M8823 |
| | | RRID: AB_2637089 |
| Brilliant Violet 711^{™} anti-mouse CD8a Antibody (clone 53-6.7) | Biolegend | 100747 |
| | | RRID: AB_11219594 |
| Brilliant Violet 785^{™} anti-mouse CD3 Antibody (clone 17A2) | Biolegend | 100231 |
| | | RRID: AB_11218805 |
| Brilliant Violet 650^{™} anti-mouse CD4 Antibody (clone RM4-5) | Biolegend | 100555 |
| | | RRID: AB_2562529 |
| Purified Rat Anti-Mouse IFN-γ (clone XMG1.2) | BD Biosciences | 554409 |
| | | RRID: AB_398550 |
| Brilliant Violet 421^{™} anti-mouse CD11c Antibody (clone N418) | Biolegend | 117329 |
| | | RRID: AB_10897814 |
| Brilliant Violet 650^{™} anti-mouse/human CD11b Antibody (clone M1/70) | Biolegend | 101239 |
| | | RRID: AB_11125575 |
| Alexa Fluor^{®} 700 anti-mouse I-A/I-E Antibody (clone M5/114.15.2) | Biolegend | 107621 |
| | | RRID: AB_493726 |
| FITC anti-mouse CD86 Antibody (clone PO3) | Biolegend | 105109 |
| | | RRID: AB_313162 |
| Brilliant Violet 605^{™} anti-mouse CD45 Antibody (clone 30-F11) | Biolegend | 103155 |
| | | RRID: AB_2650656 |
| Goat Anti-Mouse IgG(H+L)-HRP | Southern Biotech | 1036-05 |
| | | RRID: AB_2794348 |
| Goat Anti-Mouse IgG₁-HRP | Southern Biotech | 1071-05 |
| | | RRID: AB_2794426 |
| Goat Anti-Mouse IgG_{2c}-HRP | Southern Biotech | 1078-05 |
| | | RRID: AB_2794462 |
| | | |

| **Bacterial and Virus Strains** | | |
|---|---|---|
| NEB^{®} Turbo Competent E. coli (High Efficiency) | New England Biolabs | C2984H |
| | | |

| **Biological Samples** | | |
|---|---|---|
| | | |

| **Chemicals, Peptides, and Recombinant Proteins** | | |
|---|---|---|
| In-Fusion^{®} HD Cloning Kit | Takara Bio | 638909 |
| Fluorescein-12-UTP | Sigma-Aldrich | 11427857910 |
| N6-Methyladenosine-5'-Triphosphate | TriLink Biotechnologies | N-1013 |
| Pseudouridine-5'-Triphosphate | TriLink Biotechnologies | N-1019 |
| Inosine-5'-Triphosphate | TriLink Biotechnologies | N-1020 |
| FastAP | Thermo Fischer Scientific | EF0652 |
| RNaseR | Lucigen | RNR07250 |
| RNA Loading Dye, (2X) | New England Biolabs | B0363S |
| GeneRuler 1 kb DNA Ladder, ready-to-use | Thermo Fischer Scientific | SM0313 |
| RiboRuler Low Range RNA Ladder, ready-to-use | Thermo Fischer Scientific | SM1833 |
| T4 DNA Ligase | New England Biolabs | M0202M |
| DNase I | Ambion | AM2222 |
| T4 PNK | New England Biolabs | M0201S |
| RNA Fragmentation Reagents | Thermo Fischer Scientific | AM8740 |
| Ribolock RNase Inhibitor | Thermo Fischer Scientific | EO0382 |
| TRIzol | Thermo Fischer Scientific | 15596018 |
| Phenol:Chloroform:lsoamyl Alcohol 25:24:1 Saturated with 10 mM Tris, pH 8.0, 1 mM EDTA | Sigma-Aldrich | P2069 |
| cOmplete^{™} Protease Inhibitor Cocktail | Sigma-Aldrich | 11697498001 |
| Dynabeads^{™} Protein A for Immunoprecipitation | Thermo Fischer Scientific | 10002D |
| PEG 8000, Molecular Biology Grade (Polyethylene Glycol 8000) | Promega | V3011 |
| DAPI | Thermo Fischer Scientific | D1306 |
| Annexin V Binding Buffer, 10X concentrate | BD Biosciences | 556454 |
| Annexin V, Alexa Fluor^{™} 647 conjugate | Thermo Fischer Scientific | A23204 |
| Falcon^{®} 5mL Round Bottom Polystyrene Test Tube, with Cell Strainer Snap Cap | Corning | 352235 |
| Falcon^{®} 40 µm Cell Strainer, Blue, Sterile, | Corning | 352340 |

| **REAGENT or RESOURCE** | **SOURCE** | **IDENTIFIER** |
|---|---|---|
| Polyinosine-polycytidylic acid -TLR3 agonist | InvivoGen | vac-pic |
| EndoFit Ovalbumin | InvivoGen | vac-pova |
| Two OVA peptide standards for ELISPOT | InvivoGen | vac-sin |
| in vivo-jetPE! | Polyplus transfection | 201-10G |
| Corning^{®} Matrigel^{®} Growth Factor Reduced (GFR) Basement Membrane Matrix | Corning | 356231 |
| D-Luciferin Firefly, potassium salt | Biosynth International | L-8220 |
| Histopaque^{®}-1083 | Sigma-Aldrich | 10831 |
| BD GolgiPlug^{™} Protein Transport Inhibitor (Containing Brefeldin A) | BD Biosciences | 555029 |
| Nunc MaxiSorp^{™} flat-bottom | Thermo Fischer Scientific | 44-2404-21 |
| Bovine Serum Albumin | Sigma-Aldrich | A9418-5G |
| 1-Step^{™} Ultra TMB-ELISA Substrate Solution | Thermo Fischer Scientific | 34028 |
| Stop Solution for TMB Substrates | Thermo Fischer Scientific | N600 |
| Antibody Diluent | Thermo Fischer Scientific | 3118 |
| VECTASHIELD^{®} Antifade Mounting Medium with DAPI | Vector Laboratories | H-1200 |
| Square Cover Glasses No. 1 1/2, Corning^{®} | VWR | 89239-698 |
| Lipofectamine 3000 | Thermo Fischer Scientific | L3000008 |
| Opti-MEM^{™} I Reduced Serum Medium | Thermo Fischer Scientific | 31985088 |
| DMEM, high glucose, pyruvate | Thermo Fischer Scientific | 11995-073 |
| Trypsin-EDTA (0.25%), phenol red | Thermo Fischer Scientific | 25200056 |
| Penicillin-Streptomycin | Thermo Fischer Scientific | 15140-163 |
| HyClone Characterized Fetal Bovine Serum (FBS), U.S. Origin | Thermo Fisher Scientific | SH30071.03 |
| NuPAGE^{™} 4-12% Bis-Tris Protein Gels, 1.0 mm, 15-well | Thermo Fisher Scientific | NP0323BOX |
| NuPAGE^{™} MOPS SDS Running Buffer (20X) | Thermo Fisher Scientific | NP0001 |
| 2x Laemmli Sample Buffer | Bio-Rad Laboratories | 161-0737 |
| Phosphate Buffered Saline with Tween^{®} 20 (PBST-20X) | Cell Signaling Technologies | 9809 |
| milliTUBE 1ml AFA Fiber | Covaris | 520130 |
| RIG-I purified protein | Peisley et al., 2013 | N/A |
| K63-Ubₙ purified protein | Dong et al., 2011 | N/A |
| MAVS CARD-S purified protein | Wu et al., 2016 | N/A |
| BIOMOL^{®} Green | Enzo Life Sciences | BML-AK111-0250 |
| SNAP-Surface^{®} Alexa Fluor^{®} 647 | New England Biolabs | S9136S |
| ³⁵S-IRF3 | Ahmad et al., 2018 | N/A |
| SYBR^{™} Gold Nucleic Acid Gel Stain (10,000X Concentrate in DMSO) | Thermo Fisher Scientific | S11494 |
| | | |

| Critical Commercial Assays | | |
|---|---|---|
| ZymoPURE II Plasmid Prep Kits | Zymo Research | D4200 |
| MEGAscript^{™} T7 Transcription Kit | Thermo Fisher Scientific | AM 1334 |
| Direct-zol RNA Miniprep | Zymo Research | R2051 |
| RNeasy Mini Kit | Qiagen | 74106 |
| RNA Clean & Concentrator-5 | Zymo Research | R1013 |
| Zymoclean Gel Recovery Kit | Zymo Research | R1011 |
| RiboMinus Eukaryote System v2 kit | Thermo Fisher Scientific | A15026 |
| Poly(A)Purist^{™} MAG Kit | Thermo Fisher Scientific | AM 1922 |
| Brilliant II QRT-PCR Master Mix Kit, 1-Step | Agilent | 60080 |
| Neon^{™} Transfection System 100 µL Kit | Thermo Fisher Scientific | MPK10025 |
| RNA ScreenTape | Agilent | 5067-5576 |
| RNA ScreenTape Sample Buffer | Agilent | 5067-5577 |
| LIVE/DEAD^{™} Fixable Green Dead Cell Stain Kit, for 488 nm excitation | Thermo Fisher Scientific | L23101 |
| BD Cytofix/Cytoperm Fixation/Permeabilization Solution Kit | BD Biosciences | 554714 |
| | | |

| Deposited Data | | |
|---|---|---|
| m6A-irCLIP sequencing data | This paper | GEO: GSE116007 |
| ChIRP-MS data | Chen et al., 2017 | N/A |
| | | |

| **Experimental Models: Cell Lines** | | |
|---|---|---|
| HeLa | ATCC | CCL-2 |
| HEK293T | ATCC | CRL-3216 |
| RIG-I KO HeLa | Chen et al., 2017 | N/A |
| YTHDF2 KO HeLa | Dr. Chuan He | N/A |
| | | |

| **Experimental Models: Organisms/Strains** | | |
|---|---|---|
| **REAGENT or RESOURCE** | **SOURCE** | **IDENTIFIER** |
| C57BL/6J mice | Jackson Laboratories | 664 |
| | | |

| **Oligonucleotides** | | |
|---|---|---|
| qRT-PCR primers | This paper | Table S1 |
| irCLIP sequencing primers | Zarnegar et al., 2016 | N/A |
| SMARTpool ON-TARGETplus METTL3 siRNA | Dharmacon | L-005170-02-0005 |
| ON-TARGETplus Non-targeting Control siRNA #1 | Dharmacon | D-001810-01-05 |
| | | |

| **Recombinant DNA** | | |
|---|---|---|
| Plasmid: autocatalytic-splicing linear GFP-IRES (circGFPd2IRES) | Chen et al., 2017 | N/A |
| Plasmid: circGFPd2IRES_N'5BoxB | This paper | pRC0050 |
| Plasmid: pPB-CAG-Flag-GGS-lambda | Dr. Chuan He | N/A |
| Plasmid: pPB-CAG-Flag-YTHDF1N | Dr. Chuan He | N/A |
| Plasmid: pPB-CAG-Flag-YTHDF1N-lambda | Dr. Chuan He | N/A |
| Plasmid: pPB-CAG-Flag-YTHDF2N | Dr. Chuan He | N/A |
| Plasmid: pPB-CAG-Flag-YTHDF2N-lambda | Dr. Chuan He | N/A |
| Plasmid: pCAGGS-Flag-YTHDF1N | This paper | pRC0070 |
| Plasmid: pCAGGS-Flag-YTHDF1N-GS-lambda | This paper | pRC0071 |
| Plasmid: pCAGGS-Flag-YTHDF2N | This paper | pRC0103 |
| Plasmid: pCAGGS-Flag-YTHDF2N-GS-lambda | This paper | pRC0104 |
| Plasmid: pCAGGS-3xFlag-YTHdomain | This paper | pRC0151 |
| Plasmid: pCAGGS-3xFlag-mRuby3-YTHdomain | This paper | pRC0152 |
| Plasmid: pCAGGS-3xFlag-YTHdomain-GS-lambdaN | This paper | pRC0164 |
| Plasmid: pCAGGS-3xFlag-mRuby3-YTHdomain-GS-lambdaN | This paper | pRC0165 |
| | | |

| **Software and Algorithms** | | |
|---|---|---|
| ZEN (blue edition) | Carl Zeiss Microscopy | zeiss.com/microsc opy/us/products/mi croscope-software/zen/.html |
| FlowJo_V10 | FlowJo, LLC | flowjo.com/solution s/flowjo/downloads |

### YTHDF2 Rescue and YTHDF1/2 Tethering to CircBoxB

As described above, plasmids expressing YTHDF1N or YTHDF2N with and without a lambda peptide (λN) (i.e., a BoxB-binding protein), were electroporated into cells via the NEON^{™} Transfection System. After 12 hours, cells were passaged and plated such that 24 hours later they would be 70 to 80% confluent. 24 hours after this, 500 ng of circBoxB (circRNA with 5 BoxB sites) was transfected with Lipofectamine 3000. RNA was harvested and qRT-PCR was performed with Brilliant II SYBR Green qRT-PCR Master Mix and a LightCycler 480 as described above. Extra duplicates were set aside for protein lysate collection and ectopic protein expression in these conditions was simultaneously confirmed via Western blot.

### RNA Immunoprecipitation-qPCR

Plasmids expressing Flag-tagged YTHDF1 N or YTHDF2N with and without a λN were electroporated into cells via the NEON^{™} Transfection System, then later passaged into 6-well format in a timeline described above. Approximately 3 million cells were harvested with 0.25% Trypsin-EDTA (Thermo Fisher Scientific, 25200056), then washed with PBS. Cells were then lysed in cell lysis buffer (50 mM Tris pH 8.0, 100 mM NaCl, 5 mM EDTA, 0.5% NP-40 with proteinase inhibitor) by Covaris Ultrasonicator with the following settings: Fill Level 10, Duty Cycle 5%, Peak Incident Power 140 W, Cycles/Burst 200, time per tube 300s. Cell lysate was pelleted for 15 minutes at 16,000 rcf. Supernatant was collected and incubated with 100 µL of Anti-FLAG^{®} M2 magnetic beads (Sigma-Aldrich, St. Louis, MO) for two hours rotating at room temperature to pull down YTHDF1N or YTHDF2N. Beads were washed three times with cell lysis buffer and one time with PBS. Beads were resuspended in 500 µL of TRIZOL^{®} and total RNA was extracted using an RNEASY^{®} Mini kit (Qiagen, 74106). qRT-PCR was performed with Brilliant II SYBR Green qRT-PCR Master Mix and a LightCycler 480 as described above. RNA levels were normalized as percent of input within each biological replicate. Results were presented as the fold change of the enrichment of circRNA over actin.

### FACS Analysis

Cells were seeded into 24-well format at 60,000 cells per well in DMEM with FBS without antibiotics. After 24 hours, cells were transfected with siRNA per the manufacturer's recommendations. DHARMAFECT^{®} SMARTpool ON-TARGETplus METTL3 siRNA (Dharmacon, L-005170-02-0005) was used as the knockdown siRNA and ON-TARGETplus Non-targeting control siRNAs (Dharmacon, D-001810-01-05) were used as the non-targeting siRNA. Media was refreshed at 12 and 36 hours following transfection. 48 hours after transfection, cells were collected via 0.25% Trypsin-EDTA and stained with Annexin V-647 (Thermo Fisher Scientific, A23204) in Annexin binding buffer for 15 minutes. Cells were then spun down and re-suspended into DAPI in Annexin binding buffer (BD Biosciences, 556454) for 5 minutes. Cells were resuspended in Annexin binding buffer without stain and passed through round-bottom tubes with cell strainer cap (Corning, 352235). Flow analysis was done on a special order FACS Aria II (BD Biosciences). Cells subject to the same transfection as above were collected and protein lysate was collected. METTL3 knockdown was confirmed via western blot using anti-METTL3 antibody.

### immunization of Mice

Eight-to-twelve-week-old female C57BL/6 mice purchased from Jackson Laboratories were immunized subcutaneously at the base of the tail with 100 µg per mouse of OVA (Invivogen, vac-pova) adjuvanted with 25 µg of HMW vaccine grade Poly I:C (Invivogen, vac-pic), 25 µg of circular RNA alone or with *in* vivo-jetPEI (Polyplus Transfection, 201-10G), 25 µg of modified RNA alone or with *in vivo-Jet* PEI. PEI/RNA complexes were formulated as per the manufacturer's instructions. Mice were bled via the lateral tail or facial vein at regular intervals for analysis of CD8+ T cell and antibody responses after vaccination as indicated in the figures. A booster vaccination after 5 weeks of primary vaccination was given where indicated. For tumor establishment and proliferation studies, 0.5 million OVA-expressing B16 melanoma cells with matrigel were delivered in the right and left flanks of mice fourteen days after a single RNA vaccination. Tumors were measured twice a week and bioluminescence was measured once a week. Bioluminescence was measured by injecting 3 mg per 20 g mouse of D-luciferin intraperitoneally and imaged at 20 seconds to 1 minute range of exposure using an Ami HT imager (Spectral Instruments). All animal procedures were performed in accordance with guidelines established by Stanford university institutional animal care and use committee guidelines.

### CD8+ T-cell Assay

Primary and memory CD8+ T-cell responses were evaluated at day 7 after primary and secondary immunizations. Briefly, peripheral blood mononuclear cells (PBMCs) were enriched using a sucrose density gradient separation (Histopaque, 1083; Sigma Aldrich 10831) and cultured with OVA-specific MHC class I restricted peptide at 1 pg/mL (SIINFEKL) (Invivogen, vac-sin) for restimulation *ex-vivo* in the presence of BD Golgi Plug TM for 5 hours. Stimulated cells were first stained for surface markers anti-mouse CD8α (Biolegend, clone 53-6.7), anti-mouse-CD3 (Biolegend, clone 17A2), anti-mouse CD4 (Biolegend, clone RM4-5) followed by fixation in BD cytofix/cytoperm and intracellular staining with anti-mouse IFN-γ (BD Bioscience, clone XMG1.2), in BD cytoperm buffer. Dead cell was excluded using live/dead aqua stain (Invitrogen). Labeled cells were acquired on a FACS LSR-II cytometer and data were analyzed using Flow JO software (TreeStar).

### Antibody ELISA

Ninety-six-well plates (Nunc MaxiSorp, 442404-21) were coated with 100 µl of 20 µg/mL of OVA protein (Invivogen) overnight at 4 °C. Plates were washed 3 times with PBS/0.5% Tween-20 using a Bio-Rad auto plate washer and blocked with 200 µl of 4% BSA (Sigma Aldrich) for 2 hours at room temperature. Serum samples from immunized mice at the indicated time points were serially diluted in 0.1% BSA in PBS/0.5% Tween-20 and incubated on blocked plates for 2 hours at room temperature. Wells were washed and incubated with anti-mouse IgG-HRP (1:5000), anti-mouse IgG1-HRP conjugate (1:5000) and anti-mouse IgG2c-HRP conjugate (1:2000) in PBS/0.5% Tween-20 for 2 hours at room temperature. Detection antibodies were obtained from Southern Biotech. Plates were washed and developed using 100 µl per well of tetramethylbenzidine (TMB) substrate (Thermo Fisher Scientific, 34028), followed by stopping the reaction using stop solution (Thermo Fisher Scientific, N600). Plates were analyzed using a Bio-Rad plate reading spectrophotometer at 450 nm with correction at 595 nm. Antibody titers were represented as serum reciprocal dilution yielding a >0.3 optical density (OD) value at 450 nm.

### RIG-I ATPase Assay

0.1 µM RIG-I was pre-incubated with the specified circular RNA or 512 bp 5' ppp dsRNA (0.4 ng/µl) in buffer B (20 mM HEPES pH 7.5, 150 mM NaCl, 1.5 mM MgCl2). The reaction was initiated by adding 2 mM ATP at 37 °C. Aliquots (10 µl) were withdrawn at 2, 4, or 8 minutes after ATP addition, and immediately quenched with 100 mM EDTA. The ATP hydrolysis activity was measured using GREEN^{™} Reagent (Enzo Life Sciences). The GREEN^{™} Reagent (90 µl) was added to the quenched reaction at a ratio of 9:1, and OD₆₅₀ was measured using a SYNERGY^{™} 2 plate reader (BioTek).

### RIG-I Native Gel Shift Assay

RNA (1 ng/> L) was incubated with RIG-I (500nM) in buffer A (20 mM HEPES pH 7.5, 50 mM NaCl, 1.5 mM MgCl2, 2 mM ATP, and 5 mM DTT) at room temperature for 15 minutes. Poly-ubiquitin was then added at the indicated concentration and incubated at room temperature for 5 minutes. The complex was analyzed on Bis-Tris native PAGE gel (Life Technologies) and was stained with SYBR^{®} Gold stain (Life Technologies). SYBR^{®} Gold fluorescence was recorded using the scanner FLA9000 (Fuji) and analyzed with Multigauge (GE Healthcare).

### RIG-I Polymerization Assay

0.4 µM RIG-I was incubated with the specified circular RNAs (1 ng/µl) in buffer A (20 mM HEPES pH 7.5, 50 mM NaCl, 1.5 mM MgCl2, 2 mM ATP, and 5 mM DTT) at room temperature for 15 minutes. Prepared samples were adsorbed to carbon-coated grids (Ted Pella) and stained with 0.75% uranyl formate. Images were collected using a TECNAI^{™} G2 Spirit BioTWIN transmission electron microscope at 30,000x or 49,000x magnification.

### Protein Preparation

Human RIG-I was expressed as previously reported (Peisley et al., 2013). Briefly, the proteins were expressed in BL21(DE3) at 20°C for 16-20 hours following induction with 0.5 mM IPTG. Cells were homogenized using an Emulsiflex C3 (Avestin), and the protein was purified using a three-step protocol including Ni-NTA, heparin affinity chromatography, and size exclusion chromatography (SEC) in 20 mM HEPES, pH 7.5, 150 mM NaCl and 2 mM DTT.

K63-Ubn was synthesized as previously reported (Dong et al., 2011). Briefly, mouse E1, human Ubc13, Uev1a, and ubiquitin were purified from BL21(DE3) cells, and were mixed in a reaction containing 0.4 mM ubiquitin, 4 µM mE1, 20 µM Ubc13 and 20 µM Uev1a in a buffer (10 mM ATP, 50 mM Tris pH 7.5, 10 mM MgCl2, 0.6 mM DTT). After incubating the reaction overnight at 37 °C, synthesized K63-Ubn chains were diluted 5-fold into 50 mM ammonium acetate pH 4.5, 0.1 M NaCl and separated over a 45 mL 0.1-0.6 M NaCl gradient in 50 mM ammonium acetate pH 4.5 using a Hi-Trap SP FF column (GE Healthcare). High molecular weight fractions were applied to an S200 10/300 column equilibrated in 20 mM HEPES pH 7.5, 0.15 M NaCl.

MAVS CARD was expressed as a fusion construct with the SNAP tag (CARD-S) in BL21(DE3) cells at 20°C for 16-20 hours following induction with 0.4 mM IPTG. The SNAP tag allows fluorescent labeling of MAVS CARD. MAVS CARD-S fusion was purified using Ni-NTA affinity chromatography as described (Wu et al., 2016), with the exception of using 0.05% NP-40 instead of CHAPS. Purified CARD-S was denatured in 6 M guanidinium hydrochloride for 30 minutes at 37 °C with constant shaking, followed by dialysis against refolding buffer (20 mM Tris, pH 7.5, 500 mM NaCl, 0.5 mM EDTA and 20 mM BME) at 4 °C for 1 hour. Refolded CARD-S was passed through a 0.1µ filter and subsequently fluorescently labeled with Alexa647-benzylguanine (NEB) on ice for 15 minutes according to the manufacturer's instructions. The labeled MAVS CARD-S was immediately used for a polymerization assay (described below).

### MAVS Polymerization Assay

The MAVS filament formation assay was performed as previously reported (Wu et al., 2013). MAVS CARD fused to SNAP (CARD-S) was labeled with BG-Alexa 647 (New England Biolabs) on ice for 15 minutes. RIG-I (1 µM) was pre-incubated with various concentrations of RNA and 2 mM ATP in the presence or absence of 6 µM K63-Ubn (in 20 mM HEPES pH 7.5, 150 mM NaCl, 1.5 mM MgCl2, 2 mM DTT) for 15 minutes at room temperature. Subsequently, labeled monomeric MAVS CARD-S (10 µM) was added to the mixture and further incubated for 1 hour at room temperature. MAVS filament formation was detected by native PAGE analysis or by negative-stain EM. Prior to running on Bis-Tris gel (Life Technologies Corp.), all the samples were subjected to one round of freeze-thaw cycle by incubating on dry ice for 5 minutes followed by incubation at room temperature for 5 minutes. Fluorescent gel images were scanned using an FLA9000 scanner (Fuji). Samples from MAVS polymerization assay were adsorbed to carbon-coated grids (Ted Pella) and stained with 0.75% uranyl formate as described previously (Ohi et al., 2004). Images were collected using a TECNAI^{™} G2 Spirit BioTWIN transmission electron microscope at 9,300x magnification.

### Immunofluorescence and Quantification

FITC-labeled RNA was synthesized as described above, except for the substitution of 5% fluorescein 12 UTP (Thermo Fisher Scientific, 11427857910) for 100% UTP in the *in vitro* transcription reaction mix. 10% m⁶A FITC-labeled RNA was synthesized with the additional substitution of 10% m⁶A for 100% ATP in the *in vitro* transcription reaction mix. RNaseR and FASTAP^{™} treatment were carried out as described. RNA quality was assessed via Tapestation.

HeLa cells were seeded on 22x22mm #1.5 thickness cover slips in 6-well format. After 12 hours, transient transfection of FITC-labeled circRNA was performed with Lipofectamine 3000 (Thermo Fisher Scientific, L3000015). After 12 hours, cells were fixed with 1% formaldehyde in PBS (Thermo Fisher Scientific, 28908) for 10 minutes at room temperature. The formaldehyde-fixed slide was rinsed in PBS and permeabilized in 0.5% Triton X-100 in PBS for 10 min at room temperature. After the permeabilization solution was rinsed, the slide was blocked with antibody diluent (Thermo Fisher Scientific, 003118) for 1 hour at room temperature. Anti-RIG-I rabbit polyclonal primary antibody (Cell Signaling Technology, 3743S) and anti-Ub-K63 mouse monoclonal antibody (eBioscience, 14-6077-82) were diluted at 1:200 in antibody diluent and incubated overnight at 4°C. After washing with PBS, slides were incubated with goat anti-rabbit IgG highly cross-adsorbed-Alexa594 (Thermo Fisher Scientific, A-11037) and goat anti-mouse IgG highly cross-adsorbed Alexa647 (Thermo Fisher Scientific, A-21236) diluted at 1:1000 in the antibody diluent for 2 hours at room temperature. The slides were washed with PBS, mounted using VECTASHIELD^{®} with DAPI (Vector Labs, H-1200) and imaged with a Zeiss LSM 880 confocal microscope (Stanford Microscopy Facility). Colocalization of RIG-I and K63-polyUb were counted if foci were directly overlapping with FITC-circRNA and/or each other.

Anti-RIG-I rabbit polyclonal primary antibody (Cell Signaling Technology, 3743S) and anti-YTHDF2 mouse polyclonal antibody (USBiological, 135486) were diluted at 1:200 each in antibody diluent. The remaining immunofluorescence steps, including secondary staining, mounting, and imaging, were performed as detailed above. Colocalization of RIG-I and YTHDF2 were counted if foci were directly overlapping with FITC-circRNA and/or each other.

### IRF3 Dimerization Assay

The dimerization assay was performed as described previously (Ahmad et al., Cell, 172: 797-810; e713 (2018)). Briefly, HEK293T cells were homogenized in hypotonic buffer (10 mM Tris pH 7.5, 10 mM KCI, 0.5 mM EGTA, 1.5 mM MgCl2, 1 mM sodium orthovanadate, 1X mammalian Protease Arrest (GBiosciences)) and centrifuged at 1000g for 5 minutes to pellet the nuclei. The supernatant (S1), containing the cytosolic and mitochondrial fractions, was used for the *in vitro* IRF3 dimerization assay. The stimulation mix containing 10 ng/µl RIG-I and 2.5 ng/µl K63-Ubn, along with the indicated amounts of RNA, was pre-incubated at 4°C for 30 minutes in 20 mM HEPES pH 7.4, 4 mM MgCl2 and 2 mM ATP. 35S-IRF3 was prepared by *in vitro* translation using T7 TNT^{®} Coupled Reticulocyte Lysate System (Promega) according to the manufacturer's instructions. The IRF3 activation reaction was initiated by adding 1.5 µl of pre-incubated stimulation mix to a 15 µl reaction containing 10 µg/µl of S1, 0.5 µl 35S-IRF3 (in 20 mM HEPES pH 7.4, 4 mM MgCl2 and 2 mM ATP) and incubated at 30 °C for 1 hour. Subsequently, the samples were centrifuged at 18,000g for 5 minutes and the supernatant was subjected to native PAGE analysis. IRF3 dimerization was visualized by autoradiography and phosphorimaging (Fuji, FLA9000).

### Dendritic Cell Activation

Mice were immunized with PBS (control) or circular RNA (25 > g/mice) subcutaneously at base of the tail. 24 hours after the immunization mice were euthanized and skin draining inguinal lymph nodes were excised. Skin draining inguinal lymph nodes were gently busted with a 3 mL syringe plunger thumb rest, and digested with 1 mg/mL collagenase type 4 for 20-25 minutes at 37 °C.

Reactions were stopped with 2 mM EDTA and single cell suspension were prepared by passing through 40 µm cell strainers.

### Statistical Analyses

All statistical analysis was performed with the software GraphPad Prism (GraphPad Software, La Jolla, CA). Student's t-, Kruskal-Wallis, or Anova-Tukey test was used whenever appropriate, p values less than 0.05 were considered statistically significant.

### EXAMPLE 1

This example demonstrates *in vitro* production and characterization of immunogenic circRNA.

A circularized Green Fluorescent Protein (GFP) mRNA containing a permuted td intron from T4 bacteriophage, termed "circFOREIGN" hereafter, is highly immunogenic in cultured mammalian cells (Chen et al., *supra*)*.* TD introns program autocatalytic splicing during *in vitro* transcription to form circFOREIGN. Prolonged treatment (>2 hours) of circFOREIGN with exonuclease RNase R degrades linear RNA byproducts and yields enriched circRNAs (Chen et al., *supra*)*.* Subsequent alkaline phosphatase treatment removes 5' phosphate from free ends. Delivery of foreign circRNAs into mammalian cells potently stimulates immune gene expression and protected against subsequent viral infection (Chen et al., *supra*)*.* A recent report suggests that exogenous circRNAs are not immunostimulatory, but 5' triphosphorylated linear RNA contaminants due to incomplete RNase R digestion trigger an immune response (Wesselhoeft et al., Mol Cell., 74(3): 508-520 (2019)). Wesselhoeft et al. used a short (30 min) RNase R treatment, and then performed HPLC to remove linear RNA from circRNA. It was previously shown that the immune stimulation by circFOREIGN synthesized *in vitro* and treated with RNase R for two hours is comparable to the circFOREIGN treated with a second round of phosphatase to remove triphosphates on contaminating linear RNA, whereas linear RNA with phosphatase treatment greatly reduces immune activation (Chen et al., *supra*)*.* Thus, circFOREIGN stimulation is independent of the presence of aberrant 5' triphosphates in the sample. However, to confirm that 5' triphosphates are not stimulating immune gene expression, all circFOREIGN molecules described herein were synthesized in the presence of phosphatase.

It was investigated whether gel purification of the circFOREIGN treated with RNase R altered the circFOREIGN immune stimulation. It was hypothesized that if there are contaminating linear RNA components that are contributing to the circFOREIGN's immunogenicity, then gel extraction would eliminate these contaminants, which have different molecular weights. The nicked-circRNA products in the gel are not immunostimulatory, since they become linear. To this end, gel purified circFOREIGN treated with RNase R and alkaline phosphate were compared to the same circFOREIGN preparation that underwent gel purification. Each RNA preparation was transfected into HeLa cells, followed by qRT-PCR analysis of innate immunity genes 24 hours later. The gel purified circFOREIGN stimulated innate immune genes with nearly the same potency (-80-90% activity) as compared to the RNase R-only circRNA (Figures 1A and 1B).

The synthesized circRNA treated with RNase R also was subjected to HPLC fractionation. Size exclusion chromatography resolved the RNase-R-treated circRNA into two fractions (Figure 1C). Concentration and TapeStation analysis of each fraction reflected that the HPLC peak 1 mirrors the results from gel electrophoresis of RNase R-treated circFOREIGN (Figure 1C), while peak 2 was degraded RNA. The resulting HPLC purification chromatogram and fractions differed from previously reported separation (Wesselhoeft et al., *supra*) due to differences in instrumentation. Transfection of each fraction into HeLa cells followed by qRT-PCR revealed that the fraction with circRNA retained an immune response but with lower activity (Figure 1D). Although peak 2 included smaller degraded RNA and undigested introns, this fraction was not immunogenic. This result is consistent with the interpretation that phosphatase treatment throughout the sample preparation had inactivated immunogenic linear RNAs. Thus, the modest decrease in stimulation in gel purified circFOREIGN (Figure 1B) was not due to loss of these RNA species. circFOREIGN integrity was better-preserved in gel purification over HPLC purification with less degradation into smaller RNA fragments in the former, which correlated with better preservation of circFOREIGN immunogenicity.

The smaller linear RNA resulting from incomplete RNase R digestion was not responsible for circRNA immunogenicity in the preparation described above. The enzymatic purification process described above appeared to best preserve circFOREIGN integrity.

### EXAMPLE 2

This example demonstrates that circFOREIGN acts as a vaccine adjuvant *in vivo.*

CircFOREIGN has previously been shown to potently stimulate immune gene expression *in vitro* (Chen et al., supra), but its behavior *in vivo* is not known. It was hypothesized that circFOREIGN has the potential to activate innate immunity and thus act as a vaccine adjuvant to increase the efficacy of the vaccine. CircFOREIGN was *in vitro* transcribed, purified, and delivered in combination with chick ovalbumin (OVA) into C57BL/6J mice by subcutaneous injection. Polyl:C served as a positive control for RNA adjuvant. CircFOREIGN was delivered as naked RNA or after packaging in the transfection agent polyethylenimine (PEl). T cells were collected and intracellular cytokine staining (ICS) was performed seven days following primary or secondary vaccinations. Serum also was collected and antibody responses were measured five weeks after vaccinations (Figure 2A). The antibodies measured are shown in Table 3.

**Table 3**

| **Antibody** | **Clone** | **Fluorochrome** |
|---|---|---|
| CD11c | N418 | BV421 |
| CD11b | M1/70 | BV650 |
| CD103 | 2E7 | PE/BB710 |
| MHCII | M5/114.15. | Alexa 700 |
| CD86 | PO3 | FITC |
| CD45 | 30-F11 | BV610 |

Induction of OVA-specific, interferon gamma-positive (Ifny+) activated CD8 T cells required adjuvant such as polyl:C, as expected (Figure 2B and Figures 3A-3C). Notably, co-injection of circFOREIGN induced potent anti-OVA T cells comparable to levels induced by polyl:C, either with naked circRNA (p=0.0088 compared to mock, Anova-Tukey's test) or in PEI nanoparticles (p=0.0039 compared to mock, Anova-Tukey's test, Figure 2B). Measurement of OVA-specific antibodies revealed that circFOREIGN alone stimulates antibody production to levels comparable to the positive control polyl:C (Figures 2C and 3B). CircFOREIGN did not require a transfection reagent in order for stimulation of OVA-specific CD8+ T cells and antibodies. In fact, the CD8+ T cell responses were higher in injections without PE!, and PEI was omitted in subsequent experiments.

After immunization of mice with circFOREIGN or control, dendritic cells (DCs) were isolated from draining lymph nodes. circFOREIGN adjuvant activated both cDC1 and cDC2 subsets, as judged by increased cell surface expression of the costimulatory molecule CD86 over control (Figures 3D and 3E).

These results provide direct *in vivo* evidence that circRNA inoculation activated DCs. DC activation can in principle facilitate antigen cross presentation and activation of CD4+ T follicular helper (fh) cells and CD8+ T cells. However, circRNA may also directly affect T cells and other immune cell types.

### EXAMPLE 3

This example demonstrates that circFOREIGN can induce anti-tumor immunity.

Because the delivery of circFOREIGN induces CD8+ T cell responses, it was hypothesized that mice exposed to circFOREIGN and OVA would have adaptive immunity against OVA-expressing tumors. Thus, mice were vaccinated with circFOREIGN and OVA and two weeks later, OVA-expressing B16 melanoma cells were implanted into the right and left flanks of the mice (Figure 2D). The OVA-B16 melanoma model is immune restricted largely through CD8+ effector T cells (Budhu et al., J Exp Med., 207(1): 223-35 (2010)). Mice receiving circFOREIGN exhibited lower tumor growth compared to negative control mice receiving PBS (Figures 2E, 2F, and 3F). The left and right tumors in each mouse correlated with each other, demonstrating that there was a systemic-wide effect from the vaccination. The mice that were vaccinated with circFOREIGN only once exhibited nearly doubled overall survival compared to the negative control mice (p=0.0173, log-rank test, Figure 2G), and were comparable to the mice receiving positive control polyl:C HMW (Figure 3G).

The results of this example indicate that circRNA immunity can be harnessed toward potential therapeutic ends.

### EXAMPLE 4

This example demonstrates that endogenous circRNA associates with m⁶A machinery.

Given that mammalian cells have endogenous circRNAs, their immune response to circFOREIGN suggests that they distinguish between self and non-self circRNAs. As discussed above, circRNAs are produced through back-splicing to covalently join the 3' and 5^{'} ends of RNA exons. Because intron identity dictates circRNA immunity (Chen et al., *supra*) but is not part of the final circRNA product, it was hypothesized that introns may direct the deposition of one or more covalent chemical marks onto the circRNA.

CircZKSCAN1 is a human circRNA produced by its endogenous introns and is not immunogenic when expressed in human cells. ZKSCAN1 introns were used to program the production of circGFP, termed "circSELF." DNA plasmids encoding circRNAs generated by protein-assisted (circSELF) or autocatalytic splicing (circFOREIGN) were transfected into HeLa cells and comprehensive identification of RNA binding proteins was performed by mass spectrometry (ChIRP-MS) (Chen et al., *supra*)*.* Writers, readers, and erasers of covalent m⁶A modification (Roundtree et al., *supra*) were analyzed in association with circRNAs (Figure 4A). It was found that circZKSCAN1, but not circFOREIGN, is associated with components of the m⁶A writer complex, such as WTAP and VIRMA (also known as Virilizer homolog or KIAA1429) as well as the m⁶A reader proteins YTHDF2, HNRNPC, and HNRNPA2B1 (Figure 4A). Neither circRNA was associated with putative m⁶A demethylases ("erasers"), such as FTO and ALKBH5. Importantly, circSELF comprises the same circRNA sequence as circFOREIGN, but is no longer immunogenic (Chen et al., *supra*), and is associated with m⁶A writer and reader proteins (Figure 4A). Two different circRNAs (circSELF and circZKSCAN1) programmed by human introns achieve similar levels of association with m⁶A writer and reader proteins, including WTAP, VIRMA, and YTHDF2 (Figure 4B).

The results of this example suggest that m⁶A modification machinery is transferred to exonic circRNAs as a memory of the introns that mediate their biogenesis, which occurs independently of circRNA sequence.

### EXAMPLE 5

This example demonstrates that self and foreign circRNAs have different m⁶A modification patterns, and that the m⁶A modification marks circRNA as "self."

The m⁶A modification patterns of human and foreign circRNAs were defined. In human cells programmed to express the appropriate circRNAs, RNase R treatment was used to enrich for circRNAs, and m⁶A-UV-C crosslinking and m⁶A immunoprecipitation (m⁶A-irCLIP) were then performed (Zarnegar et al., Nat. Meth., 13: 489-492 (2016)) to map the sites of m⁶A modification with high sensitivity (Figure 4C). m⁶A-irCLIP of circSELF vs. circFOREIGN revealed that circSELF gained m⁶A modification within 50-100 nucleotides (nt) at the 3^{'} side of the circularization junction (Figure 4D). Significant differences in modification were not observed through the rest of the transcript (Figure 5A). Because circSELF and circFOREIGN are the same exonic sequence circularized by a human (self) or phage (foreign) intron, this result indicated that human introns are sufficient to place m⁶A modification on the resulting circRNA. Moreover, comparison of endogenous circRNAs subjected to m⁶A-irCLIP to model human-programmed circRNA indicated that both have similar patterns of m⁶A modification (Figure 4E). m⁶A is enriched in the +40-100 nt window 3' of the back-splice junction on endogenous circRNAs transcriptome-wide (Figure 4E). m⁶A is known to be enriched at the last exon of linear mRNA and long non-coding RNAs (IncRNAs) (Figure 5B) (Dominissini et al., Nature, 485: 201-206 (2012); Ke et al., Genes & Development, 29: 2037-2053 (2015)); Meyer et al., Cell, 149: 1635-1646 (2012)). The finding of m⁶A modification 3' of the back-splice junction is consistent with this pattern. Splicing occurs co-transcriptionally from 5^{'} to 3^{'}, and the 3' to 5' back splice is the expected last splicing event on a circRNA (i.e. no intron remains to be spliced out).

It was then hypothesized that the chemical modification itself or in combination with the recognition of the m⁶A by RNA-binding proteins allows marking of "self' circRNA. This was tested by examining whether incorporation of m⁶A into circFOREIGN would mask the "non-self' identity and decrease the immunogenicity of the circFOREIGN. To this end, unmodified circFOREIGN or m⁶A-modified circFOREIGN were synthesized by *in vitro* transcription (Chen et al., *supra*) and circRNA was purified by RNase R treatment. Incorporation of the m⁶A modification into circRNA did not affect splicing to form circRNA and RNase R treatment enriched for circRNA (Figures 5C and 5D). The circFOREIGN was then transfected into recipient cells and anti-viral gene expression was measured. circRNA m⁶A modification in cells was concentrated at specific positions along the transcript, whereas m⁶A incorporation during *in vitro* transcription was random. Thus, all adenosines were replaced with m⁶A (100% m⁶A) or just 1% m⁶A was incorporated into the circRNA to yield an average of three m⁶A modifications for each circRNA. 100% m⁶A likely is supra-physiologic but models the consecutive occurrence of m⁶A observed *in vivo.* 1% m⁶A models the overall level of m⁶A ratio on endogenous RNA but not the modification pattern. CircFOREIGN potently induced a panel of antiviral genes, including RIG-I, MDA5, OAS, OASL, and PKR, and anti-viral gene induction was completely abrogated when all of the adenosines were replaced with m⁶A modification (Figure 6A, 100% m⁶A). 1% m⁶A incorporation significantly reduced but did not eliminate anti-viral gene induction (Figure 6A). Thus, m⁶A modification was sufficient to reduce the immunogenicity of a foreign circRNA in cultured cells.

The circFOREIGN plasmid was then modified to eliminate m⁶A consensus motifs (Dominissini et al., *supra*) by mutating all instances (n=12) of the sequences RRACH (SEQ ID NO: 17) and RRUCH (SEQ ID NO: 19) in the GFP exon. It was hypothesized that when circFOREIGN is transcribed in the nucleus of human cells, METTL3/14 may modify circFOREIGN at low levels, which would be abrogated in the ΔRRACH mutant. Plasmids encoding wild type or mutant circRNA were transfected into HeLa cells, and circRNA levels and innate immunity gene induction by qRT-PCR were quantified. The gene induction was then normalized to the level of the measured circRNA.

RRACH site mutation significantly increased circRNA induction of anti-viral genes by approximately two-fold (Figure 6B). Because m⁶A is enriched on but not exclusively present at the RRACH motif (SEQ ID NO: 18), a modified circFOREIGN plasmid was constructed where all adenosines were mutated to uracil in the GFP exons (A-less circFOREIGN, Figure 6C). Transfection of plasmids encoding the A-less circRNA led to ~100 fold-increase in anti-viral gene induction over circFOREIGN.

The results of this example provide the first evidence that specific circRNA exonic sequences impact immunity, and specifically suggest endogenous m⁶A modification dampens innate immunity.

### EXAMPLE 6

This example demonstrates that m⁶A modification of circRNA blunts vaccination response *in vivo,* and that the m⁶A reader protein YTHDF2 is necessary to mask circRNA immunity.

m⁶A modification of circRNA also decreased the immunogenicity of circRNA as adjuvant *in vivo.* When 1% m⁶A-modified circFOREIGN was used in the same adjuvant regime as unmodified circFOREIGN, 1% m⁶A modification was found to substantially reduce both the activated CD8 T cell response (Figure 6D vs. Figure 2B) and antibody titers (Figure 6E vs. Figure 2C). Repeated immunizations with 1% m⁶A-modified circFOREIGN induced an immune response that was diminished but not null (Figure 7). These results show that circFOREIGN is a potent immune stimulant *in vivo,* and that 1% m⁶A modification is sufficient to blunt circRNA immunity.

The mechanisms of m⁶A suppression of circRNA immunity were then examined. m⁶A is recognized by a family of reader proteins, the most prominent of which are the YTH-domain containing RNA binding proteins (Dominissini et al., *supra*; and Edupuganti et al., Nature Structural & Molecular Biology, 24: 870 (2017)). YTHDF2 was focused on because (i) it is the main m⁶A reader that was detected in association with endogenous circRNA or circSELF (Figures 4A and 4B), and (ii) YTHDF2 is cytoplasmic, as are endogenous and transfected circRNAs (Chen et al., *supra*; Rybak-Wolf et al., Molecular Cell, 58: 870-885 (2015); Salzman et al., PLoS ONE, 7: e30733 (2012)). CircFOREIGN transfection into YTHDF2-/- HeLa cells (Figure 8A) led to potent induction of anti-viral genes (Figure 9A). Moreover, incorporation of 1% or 10% m⁶A into circFOREIGN no longer suppressed the antiviral gene induction in YTHDF2-/- cells (Figure 9A). An independent YTHDF2-/- clone gave very similar results (Figure 8B). Furthermore, ectopic expression of YTHDF2 in YTHDF2-/- cells rescued the suppression of immune gene induction in response to m⁶A-modified circFOREIGN (Figure 9B), indicating that YTHDF2 is required for mediating the "self' identity of m⁶A-marked circRNAs.

It was next tested which domains of YTHDF2 are necessary for suppressing immune stimulation by circFOREIGN. Full-length YTHDF2 (Figure 9C) was artificially tethered to unmodified circFOREIGN and it was determined whether the proximity of m⁶A reader proteins can bypass the need for m⁶A modification to suppress circRNA immunity. Five consecutive BoxB RNA elements were introduced into circFOREIGN immediately after the splice junction, which was termed "circBoxB." Additionally, C-terminal lambdan peptide tags were cloned into proteins and expression was confirmed via western blot (Figures 8C and 8D). This allowed recruitment of YTH proteins fused to a λN peptide, as confirmed by RIP-qPCR (Figures 9C and 8E). Transfection of plasmids encoding RNA species circBoxB alone strongly stimulated anti-viral genes, and tethering of full-length YTHDF2 significantly diminished antiviral gene induction (Figure 9D).

To establish if the N-terminal domain of YTHDF2 (YTHDF2N) is sufficient for immune evasion of circFOREIGN, YTHDF2 N-terminus was tethered to unmodified circFOREIGN-BoxB. The N-terminus was not sufficient to suppress immune response to circFOREIGN (Figure 9E). Since the N-terminal domain is responsible for cellular localization of YTHDF2-RNA complex and the C-terminal domain selectively binds to m⁶A-modified RNA (Wang et al., Nature, 505: 117-120 (2014)), the C-terminal domain is likely required for diminishing antiviral gene induction by circFOREIGN.

It was then examined whether the YTH domain is capable of marking circFOREIGN as self by joining YTH to unmodified circRNA (Figure 8F). There was no significant change in RIG-I gene expression if circFOREIGN was tethered to the YTH domain or not. However, joining circFOREIGN and YTH significantly increased the expression of MDA5 and OAS1. Since full-length YTHDF2 protein is larger than each of the separate domains, the effects of tethering circFOREIGN to Red Fluorescent Protein (RFP) on cellular recognition of unmodified circRNA (Figure 8G) was tested. There was a modest decrease in stimulation of RIG-I gene expression, but none of the other tested immune sensors exhibited a change in expression. This suggests that full suppression of circFOREIGN immunogenicity requires all of the YTHDF2 domains and interaction between another protein and circRNA is not sufficient.

To test if other members of the YTH family are involved in immune suppression of circFOREIGN, the effects of tethering YTHDF1, another cytoplasmic m⁶A reader protein, to circFOREIGN using the BoxB motif was examined. The N-terminus of YTHDF1 also failed to diminish antiviral gene induction, similar to YTHDF2 (Figure 8H). Taken together, these results demonstrate that the full-length m⁶A reader protein is necessary to mask circRNA immunity and circRNA requires either the m⁶A chemical modification or m⁶A reader protein to distinguish between "self' and "foreign" circRNAs.

### EXAMPLE 7

This example demonstrates that m⁶A writer protein METTL3 is required for self/non-self recognition of circRNA.

To probe the necessity of m⁶A in conveying a "self" mark on the circRNAs, the role of METTL3, the catalytic subunit of the writer complex, for installing the m⁶A modification was investigated. Mettl3 is essential for embryonic development due to the critical role of m⁶A in timely RNA turnover (Batista et al., Cell Stem Cell, 15: 707-719 (2014)). METTL3 depletion in many human cancer cell lines leads to cell death. One possible consequence of METTL3 depletion is a deficit of m⁶A modification of endogenous circRNA, leading to immune activation. RIG-I is a RNA binding and signaling protein that senses viral RNA for immune gene activation (Wu and Hur, Current Opinion in Virology, 12: 91-98 (2015)). Foreign circRNAs have been shown to co-localize with RIG-I in human cells, and RIG-I is necessary and sufficient for circRNA immunity (Chen et al., *supra).* Thus, if m⁶A is required to prevent cells from recognizing their own circRNA as foreign and initiating an immune response, then concomitant RIG-I inactivation should ameliorate the response. Indeed, METTL3 depletion in wild-type HeLa cells led to widespread cell death, but RIG-I inactivation in HeLa cells (Chen et al., *supra*) rescued the cell death (Figure 10).

The results of this example suggest that m⁶A prevents RIG-I activation by self RNAs; however, indirect effects due to other RNA targets of METTL3 cannot be ruled out.

### EXAMPLE 8

This example demonstrates that circFOREIGN recognition by RIG-I is distinct from linear RNAs, and CircFOREIGN directly binds RIG-I and K63-polyubiquitin chain and discriminates m⁶A.

To probe the mechanism of how circRNA stimulates an innate immune response, biochemical reconstitution with purified components was employed. First, the ability of circFOREIGN to induce ATP hydrolysis by RIG-I was assessed. When RIG-I recognizes the 5' ppp dsRNA agonist, the protein's helicase domain hydrolyzes ATP (Hornung et al., Science, 314: 994-997 (2006); Schlee et al., Immunity, 31: 25-34 (2009)). Exposure of RIG-I to circFOREIGN or 5^{'} hydroxyl linear RNA did not stimulate its ATPase activity, whereas a 512 base pair 5'-triphosphate dsRNA induced ATP hydrolysis by RIG-I (Figure 11A). Next, the ability of circFOREIGN to activate purified RIG-I was tested by forming filaments directly on circFOREIGN. Electron microscopy imaging of RIG-I, circFOREIGN, and ATP did not reveal the obvious formation of filaments, whereas the positive control 5' ppp dsRNA induced RIG-I polymerization (Figure 11B). Thus, circFOREIGN does not interact with nor activate RIG-I in the same manner as 5' ppp RNA ligands, as expected.

An alternate mechanism of RIG-I activation involves lysine 63 (K63)-linked polyubiquitin chains (K63-Ubn), which interact with and stabilize RIG-I 2CARD domain oligomers (Jiang et al., Immunity, 36: 959-973 (2012); Peisley et al., Nature, 509: 110 (2014); Zeng et al., Cell, 141: 315-330 (2010)). The ability of RIG-I to bind unmodified and m⁶A-modified circFOREIGN and the dependency of the interaction on K63-polyubiquitin chains was assessed. Using a native gel shift binding assay with purified RIG-I and circFOREIGN, RIG-I was found to bind positive control 5' ppp 162bp dsRNA both in the absence (Figure 11C, lane 2) and presence (Figure 11C, lanes 3-4) of K63-polyubiquitin. RIG-I also bound both unmodified and m⁶A-modified circFOREIGN (Figure 11C, lanes 5-16). Although K63-polyubiquitin chains do not seem to be necessary for RIG-I binding to circFOREIGN, there was greater binding of RIG-I to circFOREIGN when the concentrations of K63-polyubiquitin chains were high (Figure 11C, lane 7 vs. lane 8, lane 11 vs. lane 12, lane 15 vs. lane 16). These results suggest that RIG-I discriminates between unmodified and m⁶A-modified circRNA at the level of conformational change, rather than binding. These results also support that RIG-I binding to circRNA is different than 5' ppp dsRNA ligands.

PRRs like RIG-I and MDA5 survey many RNAs, but only selectively undergo conformational change for oligomerization upon interaction with immunogenic RNA ligands (Ahmad et al., Cell, 172(4): 797-810.e13 (2018)). Similarly, the selectivity of RIG-I for 5^{'} triphosphate (present on viral RNAs) over m7Gppp cap (present on all mRNAs) is due to conformational change rather than ligand binding (Devarkar et al., Proc Natl Acad Sci USA, 113(3): 596-601 (2016). Therefore, the ability of RIG-I to discriminate against m⁶A-modified circRNA at the level of binding vs. conformational change was evaluated.

When RIG-I is activated, oligomerized RIG-I templates the polymerization of Mitochondrial Anti-Viral Signaling protein (MAVS, also known as IPS-1, Cardif, and VISA) into filaments, creating a platform for subsequent signal transduction that culminates in the activation and dimerization of IRF3 transcription factor. Purified circFOREIGN, RIG-I, K63-polyubiquitin, and MAVS were reconstituted *in vitro,* and MAVS transition from monomer into filament was monitored by gel shift (Figure 12A) or electron microscopy (Figure 12B). Unmodified circFOREIGN strongly stimulated MAVS polymerization in a concentration-dependent manner in the presence of K63-polyubiquitin (Figure 12B). Importantly, when m⁶A modification was incorporated onto circFOREIGN at 1% or 100%, the MAVS filamentation was substantially decreased or fully abrogated, respectively (Figures 12B and 12C). In the absence of K⁶3-polyubiquitin, none of the circRNA substrates induced MAVS polymerization, indicating that polyubiquitin is necessary to stabilize activated RIG-I conformation in order for subsequent MAVS polymerization and signaling to occur (Figure 11D). Quantification of MAVS filaments by electron microscopy confirmed that unmodified circFOREIGN strongly induced MAVS filamentation, whereas m⁶A modification of circFOREIGN suppressed the ability of MAVS to oligomerize (Figures 12B and 12C).

These *in vitro* results with purified components demonstrate that unmodified circFOREIGN can directly activate RIG-I in the presence of K63-polyubiquitin and activate MAVS, in the absence of any other enzyme or RNA binding proteins. Although RIG-I binding failed to distinguish between unmodified and m⁶A-modified circRNA (Figure 11C), only unmodified circFOREIGN initiated MAVS filamentation in the presence of K63-polyubiquitin (Figures 12A-12C). These results suggest that m⁶A discrimination occurs in the MAVS monomer to filament transition and is dependent on RIG-I conformational change rather than RIG-I binding.

### EXAMPLE 9

This example demonstrates that circFOREIGN activates IRF3 dimerization.

Following MAVS filamentation, dimerization of the downstream transcription factor IRF3 completes the innate immune signaling to the genome. To test the ability of circFOREIGN to activate IRF3, a cell free assay was performed by first forming the RIG-I, RNA, K63-polyubiquitin complex, and then incubating with radio-labeled IRF3 in the presence of cellular extract (S1) containing both cytosolic and mitochondrial fractions. CircFOREIGN strongly induced IRF3 dimerization in a concentration-dependent manner, whereas m⁶A-modified circFOREIGN led to substantially less IRF3 dimerization (Figure 12D, lanes 5-7 vs. lanes 8-10). The known agonist 5' ppp 162 bp dsRNA stimulated RIG-I-mediated IRF3 dimerization better when present at a substoichiometic amount, and increased dsRNA prevented effective oligomerization of RIG-I on RNA (Figure 11D). 5'-hydroxyl linear RNA did not stimulate IRF3 dimerization, as expected of the negative control (Figure 12D, lanes 2-4).

### EXAMPLE 10

This example demonstrates that circFOREIGN requires proper complex formation prior to activation.

To understand the requirements of RIG-I oligomerization and activation, the order of addition of specific components for the *in vitro* assay was examined. 5' ppp RNA showed a more potent response when pre-incubated with RIG-I and K63-polyubiquitin prior to the supplementation of S1 lysate. However, addition of 5' ppp dsRNA after the introduction of S1 lysate resulted in a reduced, albeit significant, stimulatory activity (Figure 11E, lanes 2 and 5 vs. lanes 8 and 9). Adding K63-polyubiquitin at the S1 stage was not active since there was no difference between the presence or absence of poly-ubiquitin (Figure 11E, lanes 2 and 5 vs. lanes 10 and 11). When circFOREIGN was added to S1 cellular lysate and then mixed with RIG-I and polyubiquitin, no IRF3 dimerization activity resulted (Figure 12D, lanes 11-13). This result suggests that poly-ubiquitin needs to interact with and stabilize RIG-I in the presence of agonist circRNA first, possibly due to rapid degradation or destabilization of free K63-polyubiquitin chain in cellular lysate. Therefore, the signaling complex needs to be formed before the addition of S1 cellular lysate. Additional experiments ruled out the role of endogenous RNAs in circFOREIGN-mediated activation of IRF3 (Figure 11F).

Together, the biochemical reconstitution experiments described above demonstrated that circFOREIGN, RIG-I, and K63-Ubn form a three-component signaling-competent complex for immune signaling.

### EXAMPLE 11

This example describes the distinct localization of unmodified versus m⁶A-marked circRNAs in cells

To validate the *in vitro* assay of RIG-I directly sensing circFOREIGN, immunofluorescence microscopy was performed. HeLa cells were transfected with FITC-labeled circFOREIGN, fixed with formaldehyde, and RIG-I and K63-polyubiquitin were labeled (Figure 13A). The vast majority of circFOREIGN-FITC co-localized with both RIG-I and K63-polyubiquitin (Figure 13B, 85.5%). Interaction with unmodified circFOREIGN activated RIG-I when K63-polyubiquitin was present in the complex, which allowed for subsequent stimulation of MAVS filamentation.

Since RIG-I activation discriminates between unmodified and m⁶A-modified circRNA, it was hypothesized that YTHDF2 participates in the complex that either inhibits RIG-I activation or decreases RIG-I binding. 1% m⁶A modification was previously used on circRNA, but the m⁶A level at RRACH consensus motif (SEQ ID NO: 18) was anticipated to be much lower than 1% because the m⁶A is randomly incorporated. YTHDF2 binds m⁶A at RRACH motifs (SEQ ID NO: 18) (Dominissini et al., *supra*; Meyer et al., *supra*)*.* Thus, 10% m⁶A was incorporated into circFOREIGN for better modeling of m⁶A placement at consensus sequences. Immunofluorescence microscopy was performed with unmodified or 10% m⁶A-modified circFOREIGN, RIG-I, and YTHDF2 (Figure 13C). The percentage of circRNA colocalization with RIG-I and YTHDF2 more than doubled when m⁶A-modification was present on circFOREIGN (33.8% to 65.3%), whereas the percentage of circFOREIGN interacting with RIG-I alone decreased (Figure 13D, 61.9% to 29.3%). These results demonstrate that m⁶A modification recruits YTHDF2 to the same complex with RIG-I, and the immunofluorescence studies provide orthogonal and spatial information for the distinct fates of unmodified vs. m⁶A-modified circRNAs in cells.

Taken together, the data suggest that RIG-I recognizes foreign circRNA through a mechanism that is dependent on K63-polyubiquitin (Figure 14). Forming the complex of RIG-I, unmodified RNA, and K63-polyubiquitin triggers MAVS filamentation and IRF3 dimerization to stimulate interferon production downstream. m⁶A-modified circFOREIGN also binds RIG-I but suppresses RIG-I activation, and thus self circRNAs that carry the m⁶A modification can be safely ignored. In cells, YTHDF2 acts with m⁶A to inhibit immune signaling.

The above Examples provide *in vivo* evidence that circRNA acts as potent adjuvants to induce specific T and B cell responses. circRNA can induce both innate and adaptive immune responses and has the ability to inhibit the establishment and growth of tumors. The results suggest that human circRNAs are marked at birth, based on the introns that program their back splicing, by the covalent m⁶A modification. RIG-I discriminates between unmodified and m⁶A-modified circRNAs, and is only activated by the former. RIG-I is necessary and sufficient for innate immunity to foreign circRNA (Chen et al., *supra*) while toll-like receptors are not responsive to circRNAs (Wesslhoeft et al., *supra*)*.* In contrast, foreign circRNA lacking RNA modification is recognized by RIG-I and K63-Ubn, and m⁶A modification of foreign circRNA suffice to mark them as "self" to prevent immune activation. Modification of all adenosines, or just the adenosines in the canonical m⁶A motif RRACH (SEQ ID NO: 18), in a model circRNA substantially increased circRNA induction of anti-viral genes.

These results provide the first evidence that specific circRNA exonic sequences impact immunity, and demonstrates that endogenous m⁶A modification dampens innate immunity. m⁶A modification of 5'-triphosphate linear RNA ligands also abrogates RIG-I binding and activation (Durbin et al., mBio, 7: e00833-00816 (2016); Peisley et al., Molecular Cell, 51: 573-583 (2013)). Hence, RIG-I appears to be a general reader of circRNAs and its activation is suppressed by RNA modification, a predominant feature of eukaryotic RNAs. Both unmodified and m⁶A circRNA can bind RIG-I, but only unmodified circRNA activates RIG-I to initiate MAVS filamentation. These results suggest RIG-I conformational changes are necessary to induce MAVS filamentation. This observation is analogous to the selectivity of RIG-I for 5' triphosphate (present on viral RNAs) over m7Gppp cap (present on all mRNAs) due to conformational change rather than ligand binding (Devarkar et al., *supra*)*.* Co-crystal structure and biochemical analyses show that 5^{'} triphosphate and m7Gppp both bind RIG-I with the same affinity, but the latter triggers a distinct conformational change and causes RIG-I to filter against endogenous mRNAs and lowers ATPase activity (Devarkar et al., *supra*)*.* In living cells, YTHDF2 may inhibit the RIG-I conformational transitions necessary for downstream signaling of immune genes (Figure 13).

The above Examples systematically address the necessity, sufficiency, and domain requirements of YTHDF2-mediated suppression of circRNA immunity. The requirement of full-length YTHDF2 is consistent with a recent model that YTH-proteins recruit m⁶A-modified RNAs into phase-separated condensates via their N-terminal disordered domains, i.e. both domains are needed for higher-order RNA-protein interactions (Luo, 2018). These results extend prior knowledge about YTHDF function. Although tethering just the effector domain is sufficient to induce RNA decay or translation (Wang et al., 2015; Wang et al., 2016), the full-length protein is needed for self-foreign discrimination of circRNAs. These results suggest a double-layered system for m⁶A to both sequester and block endogenous circRNAs from activating the RIG-I antiviral pathway. In addition to YTHDF2, there may also be other sensors and receptors involved in identifying endogenous circRNAs as self.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one^{"} followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A method of producing a circular RNA molecule by *in vitro* transcription, the method comprising:
(a) providing a DNA template encoding the circular RNA molecule, ribonucleotide triphosphates, and a RNA polymerase;
(c) transcribing a linear RNA from the DNA template; and
(d) circularizing the linear DNA to form a circular RNA;
wherein the ribonucleotide triphosphates comprise N6-methyladenosine-5'-triphosphate (m⁶ATP); and
wherein the circular RNA is less immunogenic compared to a circular RNA produced using the same method but in the absence of m⁶ATP.

2. The method of claim 1, wherein at least 1% of the adenosines in the recombinant circular RNA molecule are N6-methyladenosine (m⁶A).

3. The method of claim 2, wherein at least 10% of the adenosines in the recombinant circular RNA molecule are N6-methyladenosine (m⁶A), preferably, wherein all of the adenosines in the recombinant circular RNA molecule are N6-methyladenosine (m⁶A).

4. A method of reducing the innate immunogenicity of a circular RNA molecule, wherein the method comprises:
(a) providing a circular RNA molecule that induces an innate immune response in a subject; and
(b) introducing at least one nucleoside selected from N6-methyladenosine (m⁶A), pseudouridine, and inosine into the circular RNA molecule to provide a modified circular RNA molecule having reduced innate immunogenicity.

5. The circular RNA molecule obtained by the method of claim 4 for use as a medicament, the use comprising administering the modified circular RNA to a subject.

6. The method of claim 4, wherein at least 1% of the of the circular RNA molecule contains m⁶A, pseudouridine, and/or inosine, preferably, wherein at least 10% of the circular RNA molecule contains m⁶A, pseudouridine, and/or inosine.

7. A modified circular RNA for use in a treatment of delivering a therapeutic nucleic acid to a subject, wherein N6-methyladenosine (m⁶A), pseudouridine, and/or inosine is introduced into a circular RNA molecule to provide the modified circular RNA molecule having reduced immunogenicity, as compared to the circular RNA without modification.

8. The modified circular RNA for use according to claim 7, wherein the modified circular RNA comprises the therapeutic nucleic acid and wherein the therapeutic nucleic acid comprises a sequence encoding a therapeutic polypeptide.

9. A method of delivering a substance to a cell, wherein the method comprises:
(a) generating a recombinant circular RNA molecule that comprises at least one N6-methyladenosine (m⁶A);
(b) attaching a substance to the recombinant circular RNA molecule to produce a complex comprising the recombinant circular RNA molecule attached to the substance; and
(c) contacting a cell with the complex, whereby the substance is delivered to the cell.

10. The method of claim 10, wherein the substance is a protein or peptide, preferably an antigen or an epitope, or wherein the substance is a small molecule.

11. The method of any one of claims 9-10, wherein the substance is covalently linked to the recombinant circular RNA molecule.

12. A method of sequestering an RNA-binding protein in a cell, wherein the method comprises:
(a) generating a recombinant circular RNA molecule that comprises at least one N6-methyladenosine (m⁶A) and one or more RNA-binding protein binding domains; and
(b) contacting a cell comprising the RNA-binding protein with the recombinant circular RNA molecule, whereby the RNA-binding protein binds to the one more RNA-binding protein binding domains and is sequestered in the cell.

13. The method of claim 12, wherein RNA-binding protein is aberrantly expressed in the cell, and/or wherein the RNA-binding protein is encoded by a nucleic acid sequence comprising at least one mutation, and/or wherein the RNA-binding protein is associated with a disease.

14. The method of any one of claims 12-13, wherein at least 1% of the adenosines in the recombinant circular RNA molecule are N6-methyladenosine (m⁶A), preferably, wherein at least 10% of the adenosines in the recombinant circular RNA molecule are N6-methyladenosine (m⁶A), more preferably, wherein all of the adenosines in the recombinant circular RNA molecule are N6-methyladenosine (m⁶A).

15. The method of any one of claims 12-14, wherein the recombinant RNA molecule comprises a self-splicing group I intron of the phage T4 thymidylate synthase (td) gene and at least one exon and/or wherein the recombinant circular RNA molecule comprises an internal ribosome entry site (IRES), and/or wherein the recombinant circular RNA molecule comprises between 200 nucleotides and 6,000 nucleotides, preferably wherein molecule comprises about 1,500 nucleotides.
